(19) Europäisches Patentamt
European Patent Office
Office européen des brevets

(11) EP 0 713 875 B1

(12) **EUROPÄISCHE PATENTSCHRIFT**

(45) Veröffentlichungstag und Bekanntmachung des Hinweises auf die Patenterteilung:
**21.03.2001 Patentblatt 2001/12**

(51) Int Cl.[7]: **C07D 409/12**, C07D 401/12, C07D 405/14, A61K 31/505

(21) Anmeldenummer: **95117833.4**

(22) Anmeldetag: **13.11.1995**

(54) **Neue Sulfonamide**
Sulfonamides
Sulfonamides

(84) Benannte Vertragsstaaten:
**AT BE CH DE DK ES FR GB GR IE IT LI LU MC NL PT SE**

(30) Priorität: **25.11.1994 CH 355994**
**09.10.1995 CH 284295**

(43) Veröffentlichungstag der Anmeldung:
**29.05.1996 Patentblatt 1996/22**

(73) Patentinhaber: **F. Hoffmann-La Roche AG**
**4002 Basel (CH)**

(72) Erfinder:
- **Breu, Volker**
  **D-79418 Schliengen (DE)**
- **Burri, Kaspar**
  **CH-4102 Binningen (CH)**
- **Cassal, Jean-Marie**
  **F-68100 Mulhouse (FR)**
- **Clozel, Martine**
  **F-68300 St. Louis (FR)**
- **Hirth, Georges**
  **F-68330 Huningue (FR)**
- **Löffler, Bernd-Michael**
  **D-79206 Oberrimsingen (DE)**
- **Müller, Marcel**
  **CH-4402 Frenkendorf (CH)**
- **Neidhart, Werner**
  **F-68220 Hagenthal-le-Bas (FR)**
- **Ramuz, Henri**
  **CH-4127 Birsfelden (CH)**

(74) Vertreter: **Witte, Hubert, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 526 708**

Bemerkungen:
Die Akte enthält technische Angaben, die nach dem Eingang der Anmeldung eingereicht wurden und die nicht in dieser Patentschrift enthalten sind.

EP 0 713 875 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft neue Sulfonamide und deren Verwendung als Heilmittel. Insbesondere betrifft die Erfindung neue Verbindungen der Formel

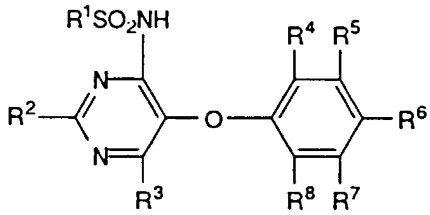

I

worin

$R^1$ ein mono- oder bicyclischer heterocyclischer Rest mit Sauerstoff, Stickstoff oder Schwefel als Heteroatom, ausgewählt aus 2-Furyl, 3-Furyl, Pyrimidinyl, 2-Pyridyl, 3-Pyridyl 4-Pyridyl, 1,2-Diazinyl, 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl und Chinazolyl; oder ein obiger heterocyclischer Rest mono-oder di-substituiert mit $C_{1-7}$-alkyl, $C_{1-7}$-alkanoyl, Halogen, amino, mono-$C_{1-7}$-alkylamino, di-$C_{1-7}$-alkylamino oder mit einem weiteren obigen heterocyclischen Rest;

$R^2$ Wasserstoff, $C_{1-7}$-Alkyl, $C_{1-7}$-Alkoxy, $C_{1-7}$-Alkylthio, $C_{1-7}$-Alkoxy-$C_{1-7}$-alkyl, $C_{1-7}$-Alkylsulfonyl-$C_{1-7}$-alkoxy, Phenyl, $C_{1-7}$-Alkyl-phenyl, $C_{1-7}$-Alkoxy-phenyl, $C_{1-7}$-Alkylendioxyphenyl, Phenyl-$C_{1-7}$-alkyl, $C_{1-7}$-Alkyl-phenyl-$C_{1-7}$-alkyl, $C_{1-7}$-Alkoxy-phenyl-$C_{1-7}$-alkyl, $C_{1-7}$-Alkylendioxyphenyl-$C_{1-7}$-alkyl, oder ein mono- oder bicyclischer heterocyclischer Rest mit Sauerstoff. Stickstoff oder Schwefel als Heteroatom, ausgewählt aus 2-Furyl, 3-Furyl, Pyrimidinyl, 2-Pyridyl, 3-Pyridyl, 4-Pyridyl, 1,2-Diazinyl, 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Thiomorpholino, Piperidino, Pyrrolidino, Benzodioxolyl, Chinolyl, Isochinolyl und Chinazolyl; oder ein obiger heterocyclischer Rest mono- oder disubstituiert mit $C_{1-7}$-alkyl, $C_{1-7}$-alkanoyl, Halogen oder mit einem weiteren obigen heterocyclischen Rest; oder Heterocyclyl-$C_{1-7}$-alkyl, wobei Heterocyclyl wie oben definiert ist;

$R^3$ $C_{1-7}$-Alkyl, $C_{1-7}$-Alkoxy, Formyl, Halogen-$C_{1-7}$-alkyl, Hydroxy-$C_{1-7}$alkyl, Amino-$C_{1-7}$-alkyl oder einen Rest $-CH_2O-A-C_{1-7}$-alkyl, $-(CH_2)_m-O-(CR^aR^b)_nOH$, $-(CH_2)_m-O-(CR^aR^b)_nOR^9$, $-(CH_2)_m-O-(CR^aR^b)_nNH_2$ oder $-(CH_2)_m-O-(CR^aR^b)_n-B-R^9$;

$R^4$-$R^8$ Wasserstoff, $C_{1-7}$-Alkoxy oder Halogen;

$R^9$ ein mono- oder bicyclischer heterocyclischer Rest mit Sauerstoff, Stickstoff oder Schwefel als Heteroatom, ausgewählt aus 2-Furyl, 3-Furyl, Pyrimidinyl, 2-Pyridyl, 3-Pyridyl, 4-Pyridyl, 1,2-Diazinyl, 1,4-Diazinyl, Morpholino, 2-Thienyl, 3-Thienyl, Isoxazolyl, Oxazolyl, Thiazolyl, Imidazolyl, Pyrrolyl, Pyrazinyl, Benzofuranyl, Benzothienyl, Indolyl, Purinyl, Chinolyl, Isochinolyl und Chinazolyl; oder ein obiger heterocyclischer Rest mono- oder di-substituiert mit $C_{1-7}$-alkyl, $C_{1-7}$-alkanoyl, Halogen oder mit einem weiteren obigen heterocyclischen Rest; oder Phenyl oder durch $C_{1-7}$-Alkyl, $C_{1-7}$-Alkoxy und/oder Halogen substituiertes Phenyl, oder $C_{1-7}$-Alkyl ;

$R^a$ und $R^b$ Wasserstoff oder $C_{1-7}$-Alkyl;

A eine ketalisierte 1,2-Dihydroxy-aethylengruppe,

B -OC(O)O-, -O(C(O)NH-, -NH(C(O)NH- oder -NHC(O)O-;

n 2, 3 oder 4; und

m 0 oder 1

bedeuten.

Der hier verwendete Ausdruck "$C_{1-7}$" bezeichnet Gruppen mit 1-7 C-Atomen, vorzugsweise 1-4 C-Atomen. Alkyl-, Alkoxy- und Alkylthiogruppen sowie Alkylgruppen als Bestandteile von Alkanoylgruppen können geradkettig oder verzweigt sein. Beispiele solcher Alkylgruppen sind Methyl, Aethyl, Propyl, Isopropyl, Butyl, sek. und tert.Butyl. Halogen bezeichnet Fluor, Chlor, Brom und Jod, wobei Chlor bevorzugt ist. Ein nieder-Alkylendioxyphenylrest ist beispielsweise ein Aethylendioxyphenylrest. Eine ketalisierte 1,2-Dihydroxyaethylengruppe ist beispielsweise die 2,2-Dimethyl-1,3-dioxolan-4,5-divl-Gruppe. Beispiele für Heterocyclylreste $R^1$ sind insbesondere substituiertes und unsubstituiertes Pyridyl, Pyrimidinyl, Thienyl und Isoxazolyl. Beispiele für Heterocyclreste $R^2$ sind insbesondere Pyrimidinyl und Morpholino. Beispiele für Heterocyclylreste $R^9$ sind insbesondere Pyridyl, Pyrimidinyl und Furyl.

Bevorzugte Verbindungen der Formel I sind solche, in denen $R^1$ ein monocyclischer S-, N-und/oder O- heterocyclischer Rest, insbesondere unsubstituiertes oder durch $C_1$-$C_7$-Alkyl, Halogen, Amino, mono- oder di-$C_1$-$C_7$-Alkylamino oder $C_1$-$C_7$-Alkanoyl substituiertes Pyridyl, Pyrimidinyl, Isoxazolyl, Furyl oder Thienyl ist. Weiterhin bevorzugt sind Verbindungen der Formel I, worin $R^2$ Wasserstoff, Pyrimidinyl, Pyridyl. Morpholino, Thiomorpholino, Piperidino, Pyrrolidino, Benzodioxolyl, $C_{1-7}$-Alkoxyphenyl oder $C_{1-7}$-Alkylthio ist und solche, $R^3$ einen Rest -O-$(CR^aR^b)_n$OH, -O-$(CR^aR^b)_n$-$NH_2$ oder ein Rest -O$(CH_2)_2$-B-$R^9$, und $R^9$ ein monocyclischer N-und/oder O-heterocyclischer Rest, insbesondere Pyridyl, Pyrazinyl oder Furyl ist.

Von besonderem Interesse sind Verbindungen der Formel I, in denen $R^1$ ein durch $C_1$-$C_7$-Alkyl substituierter Pyridylrest, $R^2$ Morpholino, $R^3$ ein Rest -O$(CH_2)_2$OC(O)NH$R^9$, $R^4$ $C_1$-$C_7$-Alkoxy und $R^5$-$R^8$ Wasserstoff sind. Bevorzugte Reste $R^9$ sind Heterocyclylreste, besonders Pyridylreste, wie 2-Pyridyl.

Die Verbindungen der oben angegebenen Formel I sind Hemmstoffe für Endothelin-Rezeptoren. Sie können daher zur Behandlung von Erkrankungen, die mit Endothelin-Aktivitäten assoziiert sind, insbesondere Kreislauferkrankungen, wie Hypertonie, Ischämie, Vasospasmen und Angina pectoris verwendet werden.

EP 0 526 708 offenbart Sulfonamid-Verbindungen als Hemmstoffe für Endothelin-Rezeptoren. Überraschend wurde nun gefunden, dass die Sulfonamide der vorliegenden Erfindung eine unerwartet hohe antagonistische Potenz in vitro aufweisen.

Die Verbindungen der Formel I können dadurch hergestellt werden, dass man

a) eine Verbindung, der Formel

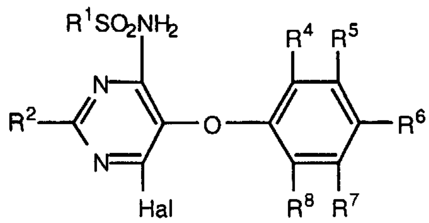

II

worin

$R^1$, $R^2$ und $R^4$ - $R^8$ die oben genannte Bedeutung haben und
Hal Halogen ist,

mit einer Verbindung der Formel

$$HO(CR^aR^b)_nXH$$

worin n, $R^a$, und $R^b$ die oben genannte Bedeutung haben und X O oder NH darstellt, umsetzt, oder

b) eine Verbindung der Formel

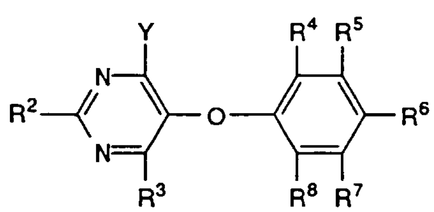

III

worin $R^2$ - $R^8$ die oben genannte Bedeutung haben,
mit einer Verbindung der Formel

$$R^1SO_2Z$$

worin $R^1$ die oben genannte Bedeutung hat und wobei Y Halogen und Z Amino, oder Y Amino und Z Halogen darstellen,
umsetzt, oder

c) eine Verbindung der Formel

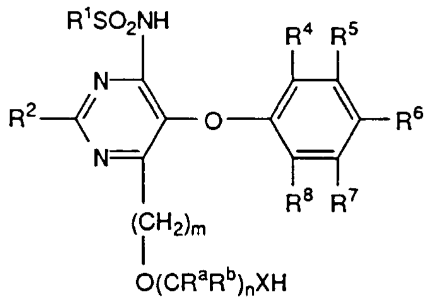

IV

worin $R^1$,$R^2$, $R^4$ - $R^8$, $R^a$, $R^b$, X, m und n die oben genannte Bedeutung haben,

c1) mit einem Isocyanat der Formel $R^9NCO$ oder einem Carbamoylchlorid der Formel $R^9NCOCl$ umsetzt, worin $R^9$ die oben genannte Bedeutung haben, oder

c2) mit Phosgen und danach mit einem Alkohol der Formel $R^9OH$; oder mit einem Chlorameisensäureester der Formel $R^9OC(O)Cl$ umsetzt; oder

d) eine Verbindung der Formel I, in der $R^3$ Halogen-$C_1$-$C_7$-alkyl darstellt, mit einer Verbindung der Formel

$$HOCH_2\text{-}A\text{-}C_1\text{-}C_7\text{-alkyl}$$

worin A eine ketalisierte 1,2-Dihydroxy-aethylengruppe darstellt,
umsetzt,

und gegebenenfalls in der erhaltenen Verbindung der Formel I enthaltene Substituenten abwandelt und/oder die erhaltene Verbindung der Formel I in ein Salz überführt.

Bei der Umsetzung einer Verbindung der Formel II mit einer Verbindung der Formel $HO(CR^aR^b)_nXH$ wird diese zweckmässig als Alkalimetallalkoholat eingesetzt. Vorzugsweise wird als Lösungsmittel das entsprechende Glykol oder der entsprechende Aminoalkohol eingesetzt, also z.B. Aethylenglykol bzw. Aminoethanol, wenn n = 2 ist. Das Alkalimetallalkoholat ist vorzugsweise Natriumalkoholat. Die Reaktion wird zweckmässig unter Erwärmen, z.B. auf 40-120°C, vorgenommen. In einer bevorzugten Ausführungsform setzt man die Verbindung $HO(CR^aR^b)_nXH$ als Mono-Natriumsalz von Aethylen-, Propylen- oder Butylenglykol bzw. Amino-ethanol, -propanol oder -butanol ein.

Die Umsetzung einer Verbindung der Formel III mit einer Verbindung der Formel $R^1SO_2Z$ kann in für die Herstellung von Sulfonamiden an sich bekannter Weise vorgenommen werden, z.B. in einem inerten organischen Lösungsmittel, wie Dimethylsulfoxid, zweckmässig unter Erwärmen und in einer Schutzgasatmosphäre, z.B. unter Argon.

Die Umsetzung gemäss Verfahrensvariante c1) kann in für die Herstellung von Carbamaten und Harnstoffen aus Alkoholen bzw. Aminen an sich bekannter Weise erfolgen. So kann eine Verbindung der Formel IV mit einem Isocyanat der Formel $R^9NCO$ in einem geeigneten wasserfreien organischen Lösungsmittel, z.B. einem Kohlenwasserstoff wie Toluol, zweckmässig unter Erwärmen, zu einer Verbindung der Formel I, in der B -OC(O)NH- ist, umgesetzt werden. Das Isocyanat kann dabei in situ, z.B. aus einem Azid der Formel $R^9CON_3$ durch thermische Zersetzung, erzeugt werden. Entsprechend können unter Verwendung von Verbindungen der Formel IV, in denen B NH ist, Verbindungen der Formel I mit B = -NHC(O)NH- erhalten werden.

Nach Verfahrensvariante c2) kann eine Verbindung der Formel IV, in der B Sauerstoff ist, mit Phosgen und danach mit einem Alkohol der Formel $R^9OH$ zu einer Verbindung der Formel I, in der A ein Rest -OC(O)O- ist, umgesetzt

werden. Anstelle von Phosgen kann ein Phosgensalz, wie Diphosgen (Cl-$COOCCl_3$) oder Triphosgen ($CO(OCCl_3)_2$ eingesetzt werden. Analog erhält man ausgehend von Verbindungen der Formel IV mit B = NH Verbindungen der Formel I mit B = -NHC(O)O-. Das Phosgen wird dabei zweckmässig als Lösung in einem inerten wasserfreien organischen Lösungsmittel, z.B. einem Kohlenwasserstoff wie Toluol, eingesetzt. Die Umsetzung mit Phosgen kann bei Raumtemperatur vorgenommen werden. Das intermediär erhaltene Säurechlorid wird unmittelbar mit dem Alkohol $R^9OH$, zweckmässig unter Erwärmen, umgesetzt.

Die Umsetzung gemäss Verfahrensvariante d) kann unter den für die Verfahrensvariante a) beschriebenen Reaktionsbedingungen durchgeführt werden und liefert Verbindungen der Formel I, in denen $R^3$ ein Rest -$CH_2$O-A-nieder-alkyl ist.

In der so erhaltenen Verbindung der Formel I können darin anwesende Substituenten abgewandelt werden. So kann eine Methylgruppe $R^3$ durch Oxidation in eine Formylgruppe umgewandelt werden. Die Oxidation kann in an sich bekannter Weise, z.B. mit Selendioxid, vorgenommen werden. In der so erhaltenen Verbindung kann die Formylgruppe zur Hydroxymethylgruppe reduziert werden. Diese Reduktion kann in an sich bekannter Weise, z.B. mittels Reduktionsmitteln wie $NaBH_4$, bewerkstelligt werden. Die Hydroxymethylgruppe kann durch Umsetzung mit einem Halogenierungsmittel, wie $POCl_3/PCl_5$ in eine Halogenmethylgruppe übergeführt werden. Weiterhin können N-heterocyclische Reste, wie Pyridyl, zu N-Oxiden oxidiert werden. Alle diese Reaktionen können nach an sich bekannten Methoden vorgenommen werden. Die Verbindungen der Formel I können in an sich bekannter Weise in Salze, z.B. Alkalisalze wie Na- und K-Salze oder Erdalkalimetallsalze, wie Ca- oder Mg-Salze überführt werden.

Die als Ausgangsmaterial eingesetzten Verbindungen können, soweit sie nicht bekannt sind oder ihre Herstellung nachstehend beschrieben ist, in Analogie zu bekannten bzw. den weiter unten beschriebenen Methoden hergestellt werden.

Die Hemmwirkung der Verbindungen der Formel I auf Endothelinrezeptoren kann mit der nachstehend beschriebenen Versuchsanordnungen gezeigt werden:

I: Hemmung der Endothelin-Bindung am recombinanten $ET_A$-Rezeptor

Eine cDNA, die für humanen $ET_A$-Rezeptor von menschlicher Plazenta kodiert, wurde kloniert (M. Adachi, Y.-Y. Yang, Y. Furuichi und C-Miyamoto, BBRC 180, 1265-1272) und im Baculovirus-Insektenzellen-System exprimiert. Baculovirus-infizierte Insektenzellen aus einem 23 l Fermenter werden 60 Stunden nach der Infektion abzentrifugiert (3 000 x g, 15 Minuten, 4°C), in Tris-Puffer (5 mM, pH 7.4, 1 mM $MgCl_2$) resuspendiert und erneut zentrifugiert. Nach erneuter Resuspension und Zentrifugation werden die Zellen in 800 ml des gleichen Puffers suspendiert und bei -120°C eingefroren. Der Aufbruch der Zellen erfolgt beim Auftauen der Suspension in diesem hypotonen Puffergemisch. Nach wiederholtem Gefrier/Auftau-Zyklus wird die Suspension homogenisiert und zentrifugiert (25 000 x g, 15 Minuten, 4°C). Nach Suspension in Tris-Puffer (75 mM, pH 7.4, 25mM $MgCl_2$, 250 mM Saccharose) werden 1 ml Aliquots (Proteingehalt ca. 3.5 mg/ml) bei -85°C aufbewahrt.

Für den Bindungs-Assay werden die eingefrorenen Membranpräparate aufgetaut und nach 10 Minuten Zentrifugieren mit 25000 g bei 20°C in Assay-Puffer (50 mM Tris-Puffer pH 7.4, enthaltend 25 mM $MnCl_2$, 1 mM EDTA und 0,5% Rinderserumalbumin) resuspendiert. 100 µl dieser Membransuspension, enthaltend 70 µg Protein werden mit 50 µl $^{125}$I-Endothelin (spezif. Aktivität 2200 Ci/mMol) in Assay-Puffer (25000 cpm, Endkonzentration 20 pM) und 100 µl Assay-Puffer, der variierende Konzentrationen der Testverbindung enthält, inkubiert. Die Inkubation wird 2 Stunden bei 20°C oder 24 Stunden bei 4°C durchgeführt. Die Trennung von freiem und Membran-gebundenen Radioliganden wird durch Filtration über Glasfaserfilter vorgenommen.

In der Tabelle 1 ist die in dieser Versuchsanordnung ermittelte Hemmwirkung von Verbindungen der Formel I als $IC_{50}$ angegeben, d.h., als Konzentration [nM] die erforderlich ist, 50% der spezifischen Bindung von $^{125}$I-Endothelin zu hemmen.

Tabelle 1

| Verbindung von Beispiel | $IC_{50}$ [nM] |
|---|---|
| 34 | 0,3 |
| 51 | 0,4 |

II. Hemmung Endothelin-induzierter Kontraktionen an isolierten Aortenringen der Ratte

Aus der Thoraxaorta von erwachsenen Wistar-Kyoto-Ratten wurden Ringe von 5 mm Länge herausgeschnitten. Das Endothel wurde durch leichtes Reiben der Innenfläche entfernt. Jeder Ring wurde bei 37°C in 10 ml Krebs-Henseleit-Lösung unter Begasung mit 95% $O_2$ und 5% $CO_2$ in ein isoliertes Bad eingetaucht. Die isometrische Spannung der Ringe wurde gemessen. Die Ringe wurden auf eine Vorspannung von 3 g gedehnt. Nach 10 Minuten Inkubation mit der Testverbindung oder Vehikel wurden kumulative Dosen von Endothelin-1 zugegeben. Die Aktivität der Testverbindung wurde ermittelt durch die zu beobachtende Rechtsverschiebung der Dosis-Wirkungskurve von Endothelin-1 in gegenwart verschiedener Konzentrationen an Antagonist. Diese Rechtsverschiebung (oder auch "dose ratio", DR) entspricht dabei dem Quotienten aus den $EC_{50}$-Werten von Endothelin-1 in Gegenwart und in Abwesenheit von Antagonist, wobei der $EC_{50}$-Wert die für eine halbmaximale Kontraktion erforderliche Endothelin-Konzentration bezeichnet.

Aus der "dose ratio" DR wurde mittels eines Computerprogramms für jede einzelne Dosis-Wirkungskurve der entsprechende $PA_2$-Wert nach untenstehender Gleichung berechnet, welcher ein Mass für die Aktivität der Testverbindung darstellt.

$$pA_2 = \log(DR\text{-}1)\text{-}\log(\text{Antagonist-Konzentration})$$

Die $EC_{50}$ von Endothelin in Abwesenheit von Testverbindungen ist 0,3 nM.

Die mit Verbindungen der Formel I so erhaltenen Werte für $pA_2$ sind in Tabelle 2 angegeben.

Tabelle 2

| Verbindung von Beispiel | Dosisverhältnis (Rechtsverschiebung) |
|---|---|
| 34 | 9,7 |
| 51 | 9,2 |

Die Verbindungen der Formel I können aufgrund ihrer Fähigkeit, die Endothelinbindung zu hemmen, als Mittel zur Behandlung von Erkrankungen verwendet werden, die mit die Vasokonstriktion erhöhenden Vorgängen assoziiert sind. Beispiele solcher Erkrankungen sind Bluthochdruck, Koronarerkrankungen, Herzinsuffizienz, renale und myocardiale Ischämie, Niereninsuffizienz, Dialyse, cerebrale Ischämie, Hirninfarkt, Migräne, subarachnoidale Hämorrhagie, Raynaud-Syndrom und pulmonärer Hochdruck. Sie können ebenfalls bei Atherosklerose, Verhinderung der Restenosierung nach Ballon-induzierter Gefässdilatation, Entzündungen, Magen- und Duodenalulcera, Ulcus cruris, Gram-negativer Sepsis, Schock, Glomerulonephritis, Nierenkolik, Glaukom, Asthma, bei der Therapie und Prophylaxe diabetischer Komplikationen und Komplikationen bei der Verabreichung von Cyclosporin, sowie anderen, mit Endothelin-Aktivitäten assoziierten Erkrankungen Anwendung finden.

Die Verbindungen der Formel I können oral, rectal, parenteral, z.B. intravenös, intramuskulär, subcutan, intrathecal oder transdermal; oder sublingual oder als ophthalmologische Zubereitung, oder als Aerosol verabreicht werden. Beispiele von Applikationsformen sind Kapseln, Tabletten, oral verabreichbare Suspensionen oder Lösungen, Suppositorien, Injektionslösungen, Augentropfen, Salben oder Spraylösungen.

Eine bevorzugte Anwendungsform ist die intravenöse, intramuskuläre oder orale Applikation. Die Dosierung, in denen die Verbindungen der Formel I in wirksamen Mengen verabreicht werden, hängen von der Art des spezifischen Wirkstoffs, dem Alter und den Bedürfnissen des Patienten und der Applikationsweise ab. Im allgemeinen kommen Dosierungen von etwa 0,1-100 mg/kg Körpergewicht pro Tag in Betracht. Die die Verbindungen der Formel I enthaltenden Präparate können inerte oder auch pharmakodynamisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Die nachstehenden Beispiele erläutern die Erfindung weiter.

<u>Beispiel 1</u>

1.29 g Na wurden bei 50°C in 50 ml Ethylenglykol gelöst. Ansschliessend gab man bei gleicher Temperatur portionsweise 3.0 g 5-tert-Butylthiophen-2-sulfonsäure 6-chloro-5-(2-methoxy-phenoxy)-2,2'-bipyrimidin-4-ylamid zu und erhitzte 4 1/2 Stunden bei 100°C. Die klare Reaktionslösung wurde auf Eis/verdünnte HCl-Lösung gegossen und es wurde 3 mal mit jeweils 0.2 l Essigsäureester extrahiert. Die organische Phase wurde 3 mal mit Wasser gewaschen,

über Natriumsulfat getrocknet und schliesslich am Rotationsverdampfer eingedampft. Man erhielt so das 5-tert-Butyl-thiophen-2-sulfonsäure 6-(2-hydroxy-ethoxy)-5-(2-methoxy-phenoxy)-2,2'-bipyrimidin-4-ylamid als hellgelben Schaum.
MS: 493 (M-SO2).

Herstellung der Ausgangsverbindung :

a) Man löste 2.0 g 5-tert-Butyl-thiophen-2-sulfonsäurechlorid in 30 ml Ethanol bei Raumtemperatur, versetzte mit 50 ml 25%iger Ammoniaklösung und erhitzte 4 1/2 Stunden am Rückfluss. Man engte am Rotationsverdampfer ein, versetzte den Rückstand mit Wasser, extrahierte mit Essigester (150 ml), trocknete über Magnesiumsulfat und engte wieder am Rotationsverdampfer ein. Man erhielt so das 5-tert-Butyl-thiophen-2-sulfonamid als weisse Kristalle. MS: 219 (M). Mit K- tert.-Butylat in Methanol wurde daraus das Kaliumsalz erhalten.

b) Man löste 3.49 g 4,6-Dichloro-5-(2-methoxy-phenoxy)-2,2'-bipyrimidin in 125 ml Dimethylsulfoxid, fügte bei Raumtemperatur 3.855 g (5-tert-Butylthiophen-2-sulfonamid)-K zu und rührte die Lösung anschliessend 20 Stunden bei Raumtemperatur. Man versetzte dann mit weiteren 1.285 g (5-tert-Butyl-thiophen-2-sulfonamid)-K und liess noch 2 Stunden bei Raumtemperatur nachreagieren. Man gab unter kräftigem Rühren 200 ml Wasser dann 200 ml Ether zum Reaktionsgemisch, wobei sich ein feiner, weisser, kristalliner Niederschlag bildete, der abgesaugt wurde. Die Kristalle wurden in verdünnter,wässriger Salzsäure suspendiert und 1/2 Stunde bei Raumtemperatur gerührt, abgenutscht und im Hochvakuum getrocknet. Man erhielt so das 5-tert-Butyl-thiophen-2-sulfonsäure 6-chloro-5-(2-methoxy-phenoxy)-2,2'-bipyrimidin-4-ylamid als weissen, kristallinen Feststoff.
MS: 523.4 (M+H).

Beispiel 2

Eine Lösung von 3.23 g 5-tert-Butyl-thiophen-2-sulfonsäure 6-(2-hydroxy-ethoxy)-5-(2-methoxy-phenoxy)-2,2'-bipyrimidin-4-ylamid , 1.71 g 2-Pyridylcarbonsäureazid und 70 mg p-Dimethylaminopyridin in Toluol (50 ml) erhitzte man 2 Stunden bei 80°C. Man entfernte das Toluol am Rotationsverdampfer und verteilte den Rückstand zwischen Methylenchlorid (0.5 l) und 1n HCl-Lösung (0.35 l). Die organische Phase wurde über Magnesiumsulfat getrocknet, das Lösungsmittel schliesslich am Rotationsverdampfer entfernt. Das Rohprodukt wurde über Kieselgel mit Methylenchlorid/MeOH (5/1) als Laufmittel chromatographiert. Man erhielt so Pyridin-2-ylcarbaminsäure 2-[6-(5-tert-butyl-thiophen-2-ylsulfonylamino)-5-(2-methoxy-phenoxy)-2,2'-bipyrimidin-4-yloxy]-ethylester als hellgelben Feststoff, der aus Methylenchlorid/MeOH umkristallisiert wurde.
MS: 678.3 (M+H).

Beispiel 3

Man löste 26 mg Natrium in 2 ml Ethanolamin bei 50°C, versetzte bei gleicher Temperatur portionsweise mit 150 mg der Verbindung aus Beispiel 1, Abschnitt b) und erhitzte die Lösung 4 Stunden bei 100°C. Anschliessend wurde auf Eis/Wasser gegossen, mit 3n HCl auf pH 6 gestellt, wobei ein hellgelber, kristalliner Feststoff ausfiel, der abgenutscht, mit Wasser gewaschen und im Hochvakuum getrocknet wurde. Man erhielt so das 5-tert-Butyl-thiophen-2-sulfonsäure 6-(2-amino-ethoxy)-5-(2-methoxy-phenoxy)-2,2'-bipyrimidin-4-ylamid als gelbe Kristalle. MS: 557.4 (M+H).

Beispiel 4

Man löste 100 mg 5-tert-Butyl-thiophen-2-sulfonsäure 6-(2-aminoethoxy)-5-(2-methoxy-phenoxy)-2,2'-bipyrimidin-4-ylamid in 10 ml Toluol, vesetzte mit 53 mg 2-Pyridylcarbonsäureazid und erhitzte die Lösung 4 Stunden bei 120°C. Das Toluol wurde am Rotationsverdampfer entfernt und der Rückstand zwischen Essigester und Wasser verteilt. Die organische Phase wurde über Magnesiumsulfat getrocknet und schliesslich am Rotationsverdampfer eingeengt. Der Rückstand wurde an Kieselgel mit Methylenchlorid/Methanol (30/1) als Laufmittel chromatographiert. Man erhielt so das 5-tert-Butyl-thiophen-2-sulfonsäure 5-(2-methoxy-phenoxy)-6-[2-(3-pyridin-2-yl-ureido)-ethoxy]-2,2'-bipyrimidin-4-ylamid als kristallinen Feststoff. MS: 677.4 (M+H).

Beispiel 5

Zu einer Lösung von 162.5 mg 4-Amino-6-methoxy-5-(2-methoxyphenoxy)-2,2'-bipyrimidin in 10 ml Tetrahydrofuran gab man bei Raumtemperatur 92 mg NaH (65%ig), rührte die Lösung 11/2 Stunden bei Raumtemperatur und fügte anschliessend bei gleicher Temperatur 162.5 mg 5-tert--Butyl-thiophen-2-sulfonylchlorid zu. Man rührte weitere 2 Stunden bei Raumtemperatur, goss auf Eis/Wasser, extrahierte mit Essigester, säuerte die wässrige Phase an und extrahierte mit Methylenchlorid. Die vereinigten, organischen Phasen wurden über Magnesiumsulfat getrocknet und am Rotationsverdampfer eingeengt. Der Rückstand wurde über Kieselgel mit Methylenchlorid/ Methanol (20/1) als Laufmittel chromatographiert. Man erhielt das 5-tert--Butyl-N-[6-methoxy-5-(2-methoxyphenoxy)-2,2'-bipyrimidin-4-yl]-thiophen-2-sulfonamid als gelbes Pulver.
MS: 463 (M-SO2).

Herstellung der Ausgangsverbindungen:

a) Man suspendierte 2.09 g 4,6-Dichloro-5-(2-methoxy-phenoxy)-2,2'-bipyrimidin (EP-A- 0 526 708) in 75 ml Ethanol und kondensierte bei -75°C 150 ml Ammoniak mit dem Steigrohr zu. Man liess die Reaktionsmischung über Nacht auf Raumtemperatur kommen, engte im Wasserstrahlvakuum ein und verteilte den Rückstand zwischen wenig Wasser und Methylenchlorid ( 500ml ). Die organische Phase wurde über Natriumsulfat getrocknet und am Rotationsverdampfer eingeengt. Der Rückstand wurde mit Ether verrührt, der entstandene Feststoff abgetrennt und im Hochvakuum getrocknet. Das 4-Amino-6-chloro-5-(2-methoxy-phenoxy)-2,2'-bipyrimidin wurde so als feines, fast weisses Pulver enthalten. MS: 329 (M).

b) Zu einer Lösung von 2.0 g 4-Amino-6-chloro-5-(2-methoxy-phenoxy)-2,2'-bipyrimidin in 200 ml Methanol fügte man bei Raumtemperatur 6.55 g Natriummethylat und erhitzte anschliessend die Lösung 32 Stunden am Rückfluss. Das Methanol wurde am Rotationsverdampfer entfernt, der Rückstand in Methylenchlorid aufgenommen und mit 1n Salzsäure gewaschen. Die organische Phase wurde über Magnesiumsulfat getrocknet, das Lösungsmittel schliesslich im Wasserstrahlvakuum entfernt. Das Rohprodukt wurde an Kieselgel mit Methylenchlorid/Methanol (10/1) als Laufmittel chromatographiert. Man erhielt 4-Amino-6-methoxy-5-(2-methoxy-phenoxy)-2,2'-bipyrimidin als zitronengelbes Pulver. MS: 325 (M).

Beispiel 6

In Analogie zu Beispiel 1 erhielt man aus Natriumglykolat und 5-Pentyl-thiophen-2-sulfonsäure 6-chloro-5-(2-methoxy-phenoxy)-2,2'-bipyrimidin-4-ylamid das 5-Pentyl-thiophen-2-sulfonsäure 6-(2-hydroxyethoxy)-5-(2-methoxy-phenoxy)-2,2'-bipyrimidin-4-ylamid als weissen Feststoff. MS: 570.3 (M+H).

Herstellung der Ausgangsverbindungen:

a) Aus 2- Pentyl-5-(t-butylsulfonamido)thiophen ( EP-A- 0 512 675) wurde mit Ethanol/konz.HCl und Salzbildung mit Kalium-tert.-butylat in Methanol das (5-n-Pentyl-thiophen-2-sulfonsäureamid)-K erhalten.

b) Analog zu Beispiel 1, Abschnitt b) erhielt man durch Umsetzung von (5-n-Pentyl-thiophen-2-sulfonsäureamid)-K und 4,6-Dichloro-5-(2-methoxyphenoxy)-2,2'-bipyrimidin das 5-Pentyl-thiophen-2-sulfonsäure 6-chloro-5-(2-methoxy-phenoxy)-2,2'-bipyrimidin-4-ylamid als weissen Feststoff. MS: 545 (M).

Beispiel 7

Analog zu Beispiel 2 erhielt man aus 2-Pyridylcarbonsäureazid und 5-Pentyl-thiophen-2-sulfonsäure 6-(2-hydroxy-ethoxy)-5-(2-methoxy-phenoxy)-2,2'-bipyrimidin-4-ylamid den Pyridin-2-ylcarbaminsäure 2-[5-(2-methoxyphenoxy)-6-(5-pentyl-thiophen-2-ylsulfonylamino)-2,2'-bipyrimidin-4-yloxy]-ethylester als weissen Feststoff. MS: 692.4 (M+H).

Beispiel 8

In Analogie zu Beispiel 1 erhielt man aus Natriumglykolat und 5-(2,2-Dimethyl-propionyl)-thiophen-2-sulfonsäure 6-chloro-5-(2-methoxyphenoxy)-2,2'-bipyrimidin-4-ylamid das 5-(2,2-Dimethyl-propionyl)-thiophen-2-sulfonsäure 6-(2-hydroxy-ethoxy)-5-(2-methoxy-phenoxy)-2,2'-bipyrimidin-4-ylamid als rosa Pulver. MS: 586.3 (M+H).

Herstellung der Ausgangsverbindung:

a) Analog zu Beispiel 1, Abschnitt b) erhielt man durch Umsetzung des Kaliumsalzes des 5-(2,2-Dimetylpropanoyl)thiophen-2-sulfonamids (Hersellung: J Org. Chem., Vol 56, 4260 ) und 4,6-Dichloro-5-(2-methoxyphenoxy)-2,2'-bipyrimidin das 5-(2,2-Dimethyl-propionyl)-thiophen-2-sulfonsäure 6-chloro-5-(2-methoxy-phenoxy)-2,2'-bipyrimidin-4-ylamid als kristallinen Feststoff. MS: 560.1 (M+H). Mit Kalium-tert.-butylat in Methanol wurde daraus das Kaliumsalz erhalten.

Beispiel 9

Analog zu Beispiel 2 erhielt man aus 2-Pyridylcarbonsäureazid und 5-(2,2-Dimethyl-propionyl)-thiophen-2-sulfonsäure 6-(2-hydroxy-ethoxy)-5-(2-methoxy-phenoxy)-2,2'-bipyrimidin-4-ylamid den gewünschten Pyridin-2-ylcarbaminsäure 2-[6-[5-(2,2-dimethylpropionyl)-thiophen-2-ylsulfonylamino]-5-(2-methoxy-phenoxy)-2,2'-bipyrimidin-4-yloxy]-ethylester als beiges Pulver. MS: 704.3 (M+H).

Beispiel 10

1.75 g Na wurden bei 50°C in 70 ml Ethylenglykol gelöst. Ansschliessend gab man bei gleicher Temperatur portionsweise 4.9 g 5-Isopropylpyridin-2-sulfonsäure 6-chloro-5-(2-methoxy-phenoxy)-2,2'-bipyrimidin-4-ylamid zu und erhitzte 4 Stunden auf 100°C. Die klare Reaktionslösung wurde auf 200 ml Wasser gegossen, mit 3n HCl auf pH 1 gebracht, die ausgefallenen gelben Kristallle wurden abgenutscht, mit Wasser, dann mit Ether gewaschen und schliesslich im Hochvakuum getrocknet. Man erhielt so das 5-Isopropyl-pyridin-2-sulfonsäure 6-(2-hydroxy-ethoxy)-5-(2-methoxyphenoxy)-2,2'-bipyrimidin-4-ylamid als gelben, kristallinen Feststoff.
MS: 537.3(M-H).

Herstellung der Ausgangsverbindungen:

a) Man lösste 4.0 g 5-Isopropylpyridin-2-sulfonamid in 40 ml MeOH, fügte bei Raumtemperatur 2.308 g Kalium-tert.-butylat zu und rührte die Lösung weitere 20 Minuten. Anschliessend wurde am Rotationsverdampfer vollständig eingeengt und das so erhaltene Kaliumsalz im Hochvakuum getrocknet.

b) Man löste 3.49 g 4,6-Dichloro-5-(2-methoxy-phenoxy)-2,2'-bipyrimidin in 125 ml Dimethylsulfoxid, fügte bei Raumtemperatur 4.7 g (5-Isopropylpyridin-2-sulfonamid)-K zu und rührte die Lösung anschliessend 20 Stunden bei Raumtemperatur. Man goss unter kräftigem Rühren auf 350 ml Wasser und 90 ml Ether, brachte unter Zugabe von 3n HCl die Lösung auf pH 1. Der weisse, kristalline Niederschlag wurde abgesaugt und mit Wasser, dann Ether gewaschen. Die Kristalle wurden in verdünnter, wässriger Salzsäure (100ml Wasser und 50 mllln HCl) suspendiert und 5 Minuten gerührt, abgenutscht und erneut mit Wasser gewaschen und im Hochvakuum getrocknet. Man erhielt so das 5-Isopropyl-pyridin-2-sulfonsäure 6-chloro-5-(2-methoxy-phenoxy)-2,2'-bipyrimidin-4-ylamid als weissen, kristallinen Feststoff.
MS: 511.3 (M-H).

Beispiel 11

Man löste 2.0 g 5-Isopropyl-pyridin-2-sulfonsäure 6-(2-hydroxy-ethoxy)-5-(2-methoxy-phenoxy)-2,2'-bipyrimidin-4-ylamid in 80 ml Toluol, versetzte mit 1.1 g 2-Pyridylcarbonsäureazid und erhitzte die Lösung anschliessend 4 Stunden bei 90°C. Man engte am Rotationsverdampfer ein und verteilte den Rückstand zwischen 1n HCl und Essigester. Die organische Phase wurde über Magnesiumsulfat getrocknet, das Lösungsmittel im Wasserstrahlvakuum entfernt und der Rückstand an Kieselgel mit Methylenchlorid/ Methanol (30/1) als Laufmittel chromatographiert. Man erhielt so den Pyridin-2-ylcarbaminsäure 2-[6-(5-isopropyl-pyridin-2-ylsulfonylamino)-5-(2-methoxy-phenoxy)-2,2'-bipyrimidin-4-yloxy]-ethylester als gelbe Kristalle.

MS: 657.3 (M+H).

Zur Herstellung des Dihydrochlorids wurde die Verbindung in Methylenchlorid gelöst und mit der entsprechenden Menge 4.4 n HCl in Ethanol bei Raumtemperatur versetzt. Die Lösung wurde am Rotationsverdampfer eingeengt, der ausgefallene, kristalline Feststoff isoliert und 4 Sunden bei 60°C im Hochvakuum getrocknet.

Beispiel 12

In Analogie zu Beispiel 4 erhielt man aus 5-Isopropyl-pyridin-2-sulfonsäure 6-(2-amino-ethoxy)-5-(2-methoxy-phenoxy)-2,2'-bipyrimidin-4-ylamid und 2-Pyridylcarbonsäureazid das gewünschte 5-Isopropyl-pyridin-2-sulfonsäure 5-(2-methoxy-phenoxy)-6-[2-(3-pyridin-2-yl-ureido)-ethoxy]-2,2'-bipyrimidin-4-ylamid als gelbe Kristalle. MS: 656.3 (M-H).

Die Ausgangsverbindung erhielt man analog zu Beispiel 3 aus Ethanolamin und der Verbindung aus Beispiel 10, Abschnitt b) als gelben Schaum. MS: 538.3 (M+H).

Beispiel 13

In Analogie zu Beispiel 10 erhielt man aus Pyridin-2-sulfonsäure 6-chloro-5-(2-methoxy-phenoxy)-2,2'-bipyrimidin-4-ylamid und Ethylenglykol das Pyridin-2-sulfonsäure 6-(2-hydroxy-ethoxy)-5-(2-methoxy-phenoxy)-2,2'-bipyrimidin-4-ylamid als weisse Kristalle. MS: 615.4 (M-H).

Herstellung der Ausgangsverbindungen:

a) Man lösste 1.7 g 2-Pyridylsulfonsäurechlorid (J Org. Chem., Vol. 54, 389) in 30 ml Ethanol, fügte unter Eiskühlung 30 ml 25 %ige Ammoniaklösung zu und erhitzte anschliessend 4 Stunden am Rückfluss. Die Reaktionslösung wurde am Rotationsverdampfer eingeengt, der Rückstand zwischen Essigester und Wasser verteilt, die organische Phase über Magnesiumsulfat getrocknet und schliesslich am Rotationsvedampfer eingeengt, wobei das 2-Pyridylsulfonsäureamid als beiger, kristalliner Feststoff ausfiel. MS: 469.2 (M-H). Mit K-tert.-Butylat in Methanol wurde dareaus das K-Salz erhalten.

b) Analog zu Beispiel 10, Abschnitt b) erhielt man durch Umsetzung von (2-Pyridylsulfonamid)-K und 4,6-Dichloro-5-(2-methoxy-phenoxy)-2,2'-bipyrimidin das Pyridin-2-sulfonsäure 6-chloro-5-(2-methoxy-phenoxy)-2,2'-bipyrimidin-4-ylamid als weisse Kristalle. MS: 495.3 (M-H).

Beispiel 14

In Analogie zu Beispiel 11 erhielt man aus Pyridin-2-sulfonsäure 6-(2-hydroxy-ethoxy)-5-(2-methoxy-phenoxy)-2,2'-bipyrimidin-4-ylamid und 2-Pyridylcarbonsäureazid den Pyridin-2-ylcarbaminsäure 2-[5-(2-methoxyphenoxy)-6-pyridin-2-ylsulfonylamino)-2,2'-bipyrimidin-4-yloxy]-ethylester als weisse Kristalle. MS: 615.4 (M-H)

Beispiel 15

In Analogie zu Beispiel 10 erhielt man aus Pyridin-3-sulfonsäure 6-chloro-5-(2-methoxy-phenoxy)-2,2'-bipyrimidin-4-ylamid und Ethylenglykol das Pyridin-3-sulfonsäure 6-(2-hydroxy-ethoxy)-5-(2-methoxy-phenoxy)-2,2'-bipyrimidin-4-ylamid als weisse Kristalle. MS: 496 (M).

Herstellung der Ausgangsverbindungen:

a) In Analogie zu Beispiel 13 Abschnitt a) erhielt man aus 3-Pyridylsulfonsäurechlorid ( J Org. Chem., Vol. 54, 389) und Ammoniak das 2-Pyridylsulfonsäureamid als weissen, kristallinen Feststoff, daraus mit Kalium-tert.-butylat in Methanol das Kaliumsalz.

b) Analog zu Beispiel 10, Abschnitt b) erhielt man durch Umsetzung von (3-Pyridylsulfonamid)-K und 4,6-Dichloro-5-(2-methoxy-phenoxy)-2,2'-bipyrimidin das gewünschte Pyridin-3-sulfonsäure 6-chloro-5-(2-methoxyphenoxy)-2,2'-bipyrimidin-4-ylamid als weisse Kristalle. MS: 470 (M).

Beispiel 16

In Analogie zu Beispiel 11 erhielt man aus Pyridin-3-sulfonsäure 6-(2-hydroxy-ethoxy)-5-(2-methoxy-phenoxy)-2,2'-bipyrimidin-4-ylamid und 2-Pyridylcarbonsäureazid den Pyridin-2-ylcarbaminsäure 2-[5-(2-methoxyphenoxy)-6-pyridin-3-ylsulfonylamino)-2,2'-bipyrimidin-4-yloxy]-ethylester als weisse Kristalle. MS: 615.4(M-H).

Beispiel 17

Man löste 213 mg 6-[2-(tert-Butyl-dimethyl-silanoxy)-ethoxy]-5-(2-chloro-5-methoxy-phenoxy)-pyrimidin-4-ylamin in 15 ml Tetrahydrofuran, vesetzte bei Raumtemperatur mit 92 mg NaH (65%ig), rührte 2 Stunden bei Raumtemperatur und gab dann portionsweise 155 mg 5-tert-Butyl-thiophen-2-sulfonylchlorid zu. Man rührte die Lösung noch 2 Stunden bei Raumtemperatur, goss in Eiswasser und extrahierte 2 mal mit insgesamt 200 ml Essigester. Nach üblicher Verarbeitung der organischen Phase wurde das silylgeschützte Rohprodukt an Kieselgel mit Methylenchlorid/Essigester (8/1) als Laufmittel chromatographiert.

Der erhaltene, bräunliche Schaum (219 mg) wurde in 15 ml Acetonitil gelöst, bei Raumtemperatur mit 1.5 ml HF-Lösung (40%ig) versetzt und 2 Stunden gerührt. Das Reaktionsgemisch wurde zwischen Essigester und halbgesättigter NaCl-Lösung verteilt und die organische Phase wie üblich verarbeitet. Das Rohprodukt wurde an Kieselgel mit Methylenchlorid/Essigester (4/1) als Laufmittel chromatographiert und aus Ether/Hexan umkristallisiert. Man erhielt so das 5-tert-Butyl-thiophen-2-sulfonsäure 5-(2-chloro-5--methoxy-phenoxy)-6-(2-hydroxy-ethoxy)-pyrimidin-4-ylamid als weisse Kristalle. MS: 449 (M-SO2)

Herstellung der Ausgangsverbindung

a) 3-Methoxyphenol wurde nach der Vorschrift von M. Julia und I. de Rosnay, Chimie Thérapeutique 5 (1969), 334 in 2-Chlor-5-methoxyphenol mit Sulfurylchlorid umgewandelt.

b) 18,2 g 2-Chlor-5-methoxyphenol wurden in 150 ml trockenem Methanol gelöst. Man gab 9,3 g MeONa zu, gefolgt von 25 g Chlormalonsäure-dimethylester. Das Reaktionsgemisch wurde während 2 Stunden bei 50°C gerührt. Nach Abdestillieren des Lösungsmittels wurde der Rückstand im Scheidetrichter zwischen Toluol und $H_2O$ verteilt und neutral gewaschen. Nach Kristallisation in Ethanol erhielt man (2-Chlor-5-methoxy)phenoxy-dimethylmalonat, weisse Kristalle mit Smp. 68-69°C.

c) 1,43 g Na wurden in 70 ml MeOH gelöst. Dann wurden 5,8 g (2-Chlor-5-methoxy)phenoxy-dimethylmalonat und 2,29 g Formamidin-acetat zugegeben; das Reaktionsgemisch wurde während 1,5 Stunden unter Rückfluss gerührt. Dann wurde das Lösungsmittel abdestilliert, der Rückstand in $H_2O$ aufgenommen, die wässrige Phase mit Essigester extrahiert, die organische Phase verworfen und die wässrige Phase mit Essigsäure auf pH 4 angesäuert, wobei das 5-(2-Chlor-5-methoxy)phenoxy-4,6(1H,5H)-pyrimidindion als weisses Pulver ausfiel. MS: m/e = 268 (M).

d) Ein Gemisch von 3,75 g 5-(2-Chlor-5-methoxy)phenoxy-4,6(1H,5H)-pyrimidindion, 5,4 g N-Ethyldiisopropylamin, 12,5 ml $POCl_3$ in 20 ml Dioxan wurde unter Rückfluss während 18 Stunden gerührt. Nach Abdestillieren der flüchtigen Komponenten, wurde der Rückstand zwischen Essigester und $H_2O$ verteilt und neutral gewaschen. Nach Abdestillieren des Lösungsmittels wurde die Verbindung an Kieselgel mit $CH_2Cl_2$ als Fliessmittel gereinigt. Nach Kristallisation aus EtOH erhielt man 4,6-Dichlor-5-(2-chlor-5-methoxy-phenoxy)-pyrimidin als weisse Kristalle mit Smp. 88-89°C.

e) In eine Lösung von 9,9 g 4,6-Dichloro-5-(2-chloro-5-methoxy-phenoxy)-pyrimidin aus Beispiel le) in 400 ml Ethanol wurden bei -78°C ca. 500 ml $NH_3$ eingeleitet. Danach wurde das Reaktionsgemisch 15 Stunden bei -78°C und 50 Stunden bei Raumtemperatur gerührt und schliesslich eingedampft. Der Rückstand wurde zwischen Aethylacetat und Wasser verteilt und die organische Phase aufgearbeitet. Man erhielt so 8,53 g 6-Chloro-5-(2-chloro-5-methoxy-phenoxy)-pyrimidin-4-ylamin als gelbe Kristalle. MS: 285 (M).

f) 8,53 g der vorstehend erhaltenen Verbindung wurden zu einer Lösung von 0,82 g Natrium in 100 ml Aethylenglykol bei 50°C gegeben. Die Lösung wurde 20 Stunden auf 100°C erwärmt, danach zwischen halbgesättigter $NH_4Cl$-Lösung und $CH_2Cl_2$ verteilt und aufgearbeitet. Man erhielt 8,3 g 2-[6-Amino-5-(2-chloro-5-methoxy-phenoxy)-4-pyrimidin-4-yloxy]-1-ethanol als weissen Feststoff, der ohne weitere Reinigung silyliert wurde. Dazu wurde

obiges Material (8,3 g) in 300 ml Methylenchlorid gelöst, mit 8,15 g Dimethylaminopyridin versetzt und schliesslich bei Raumtemperatur mit 10,05 g t-Butyldimethylchlorsilan. Die Reaktionslösung wurde 5 Stunden bei Raumtemperatur gerührt. Dann wurde filtriert, die Lösung eingeengt, der Eindampfrückstand zwischen halbgesättigter $NH_4Cl$-Lösung und Aethylacetat verteilt und die organische Phase aufgearbeitet. Anschliessende Kristallisation aus Methylenchlorid/Hexan lieferte 7 g 6-[2-(tert-Butyldimethyl-silanyloxy)-ethoxy]-5-(2-chloro-5-methoxy-phenoxy)-pyrimidin-4-ylamin. MS: 410 (M-$CH_3$).

Beispiel 18

In Analogie zu Beispiel 2 erhiet man aus 5-tert-Butyl-thiophen-2-sulfonsäure 5-(2-chloro-5--methoxy-phenoxy)-6-(2-hydroxy-ethoxy)-pyrimidin-4-ylamid und 2-Pyridylcarbonsäureazid den Pyridin-2-ylcarbaminsäure 2-[6-(5-tert-butyl-thiophen-2-ylsulfonylamino)-5-(2-chloro-5-methoxy-phenoxy)-pyrimidin-4-yloxy]-ethylester als weisse Kristalle. MS: 634.3 (M+H).

Beispiel 19

In Analogie zu Beispiel 2 erhielt man aus der Verbindung von Beispiel 17 und 4-Pyridylcarbonsäureazid den Pyridin-4-ylcarbaminsäure 2-[6-(5-tert-butyl-thiophen-2-ylsulfonylamino)-5-(2-chloro-5-methoxy-phenoxy)-pyrimidin-4-yloxy]-ethylester als weisse Kristalle. MS: 634.3 (M+H).

Beispiel 20

In Analogie zu Beispiel 17 , wobei man 6-[2-(tert-Butyl-dimethylsilanoxy)-ethoxy]-5-(2-methoxy-phenoxy)-pyrimidin-4-ylamin als Reaktionskomponente einsetzte, erhielt man das 5-tert-Butyl-thiophen-2-sulfonsäure 6-(2-hydroxy-ethoxy)-5-(2-methoxy-phenoxy)-pyrimidin-4-ylamid als weissen Feststoff, MS: 479 (M).

Beispiel 21

Zu einer Na-Glykolat-Lösung aus 1,5 ml Ethylenglykol und 46 mg Na gab man 180 mg der Verbindung aus voriger Stufe. Zur vollständigen Auflösung wurde 1 ml DMSO zugefügt. Man liess während 3 Stunden bei 90°C reagieren. Nach Abkühlen auf Raumtemperatur wurde das Reaktionsmedium mit wässriger Zitronensäure auf pH 4 angesäuert und die gebildete Verbindung wurde anschliessend mit Essigester extrahiert. Nach Abdestillieren des Essigesters wurde das N-[5-(2-Chlor-5-methoxy-phenoxy)-6-(2-hydroxy-ethoxy)-pyrimidin-4-yl]-5-isopropyl-pyridin-2-sulfonamid aus Ethanol kristallisiert. Man erhielt 175 mg weisse Kristalle, die sich bei 180°C zersetzen.

Herstellung der Ausgangsverbindung

306 mg 4,6-Dichlor-5-(2-chlor-5-methoxy-phenoxy)-pyrimidin, 320 mg 5-Isopropyl-2-pyridin-sulfonamid und 180 mg K-tert. Butylat in 2 ml DMSO gelöst wurden während 3 Stunden bei 90°C zur Reaktion gebracht. Nach Abkühlen auf Raumtemperatur wurde das Reaktionsmedium mit wässriger Zitronensäure angesäuert; die Verbindung wurde mit Essigester extrahiert und nach Abdestillieren des Lösungsmittels aus Ethanol kristallisiert. Man erhielt 250 mg N-[6-Chlor-5-(2-chlor-5-methoxy-phenoxy)-pyrimidin-4-yl]-5-isopropyl-pyridin-sulfonamid als weisse Kristalle mit Smp. 174-175°C.

Beispiel 22

100 mg N-[5-(2-Chlor-5-methoxy-phenoxy)-6-(2-hydroxy-ethoxy)-pyrimidin-4-yl]-5-isopropyl-pyridin-2-sulfonamid und 38,5 mg 2-Pyridylcarbonsäureazid wurden in 1 ml trockenem Dioxan gelöst. Die Lösung wurde während 2 Stunden bei 95°C gerührt, wobei N2 freigesetzt wurde. Nach Abdestillieren des Lösungsmittels wurde die Verbindung aus Ethanol kristallisiert. Man erhielt 115 mg Pyridin-2-yl-carbaminsäure 2-[5-(2-Chlor-5-methoxy-phenoxy)-6-(5-isopropyl-pyridin-2-ylsulfonylamino)-pyrimidin-4-yloxy]-ethylester, als weisse Kristalle mit Smp. 190-191°C.

Beispiel 23

In Analogie zu Beispiel 21 erhielt man das 5-Isopropyl-N-[6-(2-hydroxyethoxy)-5-(2-methoxy-phenoxy)-2-(3-methoxy-phenyl)-pyrimidin-4-yl]-pyridin-2-sulfonamid. Smp. 139-140°C (aus Ethanol).

Herstellung der Ausgangsverbindung

In Analogie zu Beispiel 21, zweiter Abschnitt, erhielt man 400 mg N-[6-Chlor-5-(2-methoxy-phenoxy)-2-(3-methoxy-phenyl)-pyrimidin-4-yl]-5-isopropyl-pyridin-2-sulfonamid aus 330 mg 4,6-Dichlor-2-(3-methoxyphenyl)-5-(2-methoxy-phenoxy)-pyrimidin und 420 mg (5-Isopropyl-pyridin-2-sulfonamid)-K.

Beispiel 24

In Analogie zu Beispiel 22 erhielt man aus 115 mg 5-Isopropyl-N-[6-(2-hydroxy-ethoxy)-5-(2-methoxy-phenoxy)-2-(3-methoxy-phenyl)-pyrimidin-4-yl]-pyridin-2-sulfonamid und 38,5 mg 2-Pyridyl-carbonsäureazid 120 mg Pyridin-2-yl-carbaminsäure 2-[6-(5-isopropyl-pyridin-2-ylsulfonylamino)-5-(2-methoxy-phenoxy)-2-(3-methoxy-phenyl)-pyrimidin-4-yloxy]-ethylester. Smp. 158-160°C (aus Ethanol).

Beispiel 25

a) In Analogie zu Beispiel 21 erhielt man das N-[6-Chlor-5-(2-methoxyphenoxy)-2-methylsulfanyl-pyrimidin-4-yl]-5-isopropyl-pyridin-2-sulfonamid aus 4,6-Dichlor-2-methylsulfanyl-5-(2-methoxy-phenoxy)-pyrimidin und (5-Isopropyl-pyridin-2-sulfonamid)-K. Smp. 192°C (aus Ethanol).

b) Die Verbindung wurde mit Na-Glykolat in das 5-Isopropyl-N-[6-(2-hydroxy-ethoxy)-5-(2-methoxy-phenoxy)-2-methylsulfanyl-pyrimidin-4-yl]-pyridin-2-sulfonamid umgewandelt. Smp. 76-78°C (aus EtOH).

Beispiel 26

In Analogie zu Beispiel 22 erhielt man 106 mg Pyridin-2-ylcarbaminsäure 2-[6-(5-isopropyl-pyridin-2-ylsulfonylamino)-5-(2-methoxy-phenoxy)-2-methylsulfanyl-pyrimidin-4-yloxy]-ethylester aus 100 mg 5-Isopropyl-N-[6-(2-hydroxy-ethoxy)-5-(2-methoxy-phenoxy)-2-methylsulfanyl-pyrimidin-4-yl]-pyridin-2-sulfonamid und 2-Pyridyl-carbonsäureazid. Smp. 213-214°C (aus Ethanol).

Beispiel 27

a) Aus 4,6-Dichlor-2-(1,3-benzodioxol-5-yl)-5-(2-methoxy-phenoxy)-pyrimidin und (5-Isopropyl-pyridin-2-sulfonamid)-K erhielt man das N-[6-Chlor-2-(1,3-benzodioxol-5-yl)-5-(2-methoxy-phenoxy)-pyrimidin-4-yl]-5-isopropyl-pyridin-2-sulfonamid.

b) Diese Verbindung wurde mit Na-Glykolat in N-[2-(1,3-Benzodioxol-5-yl)-6-(2-hydroxy-ethoxy)-5-(2-methoxy-phenoxy)-pyrimidin-4-yl]-5-isopropylpyridin-2-sulfonamid übergeführt. Smp. 184°C (aus EtOH).

Beispiel 28

In Analogie zu Beispiel 22 erhielt man 110 mg Pyridin-2-ylcarbaminsäure 2-[2-(1,3-benzodioxol-5-yl)-6-(5-isopropyl-pyridin-2-ylsulfonylamino)-5-(2-methoxy-phenoxy)-pyrimidin-4-yloxy]-ethylester aus 116 mg N-[2-(1,3-Benzodioxol-5-yl)-6-(2-hydroxy-ethoxy)-5-(2-methoxy-phenoxy)-pyrimidin-4-yl]-5-isopropyl-pyridin-2-sulfonamid und 2-Pyridyl-carbonsäureazid. Smp. 184°C (aus Ethanol).

Beispiel 29

a) Aus 4,6-Dichlor-2-morpholin-4-yl-pyrimidin und (5-Isopropyl-pyridin-2-sulfonamid)-K erhielt man das N-[6-Chlor-5-(2-chlor-5-methoxy-phenoxy)-2-morpholin-4-yl-pyrimidin-4-yl]-5-isopropyl-pyridin-2-sulfonamid.

b) Die Umsetzung dieser Verbindung mit Na-Glykolat lieferte das N-[5-(2-Chlor-5-methoxy-phenoxy)-6-(2-hydroxy-ethoxy)-2-morpholin-4-yl-pyrimidin-4-yl]-5-isopropyl-pyridin-2-sulfonamid. Smp. 189-190°C (aus EtOH).

Beispiel 30

In Analogie zu Beispiel 22 wurden 116 mg N-[5-(2-Chlor-5-methoxyphenoxy)-6-(2-hydroxy-ethoxy)-2-morpholin-4-yl-pyrimidin-4-yl]-5-isopropylpyridin-2-sulfonamid mit 2-Pyridyl-carbonsäureazid zum Pyridin-2-yl-carbaminsäure 2-[5-(2-chlor-5-methoxy-phenoxy)-6-(5-isopropyl-pyridin-2-ylsulfonylamino)-2-morpholin-4-ylpyrimidin-4-yloxy]-ethylester umgesetzt. Man erhielt 106 mg weisse Kristalle aus Ethanol, die sich bei 240°C zersetzen.

Beispiel 31

In Analogie zu Beispiel 21 erhielt man aus 5-Methyl-pyridin-2-sulfonsäure [6-chlor-5-(2-methoxy-phenoxy)-2,2'-bipyrimidin-4-yl]-amid und Na-Glykolat das 5-Methyl-pyridin-2-sulfonsäure 6-(2-hydroxy-ethoxy)-5-(2-methoxy-phenoxy)-2,2'-bipyrimidin-4-ylamid. Smp. 190°C (aus Ethanol).

Herstellung der Ausgangsverbindung

a) 2-Amino-5-methylpyridin wurde diazotiert und in 2-Brom-5-methylpyridin umgewandelt, nach dem Verfahren von F.H. Case (JACS 68 (1946), 2574).

b) 4,8 g dieser Verbindung wurden in 40 ml Propylenglykol mit 7,4 g Natriumhydrogensulfid bei 150°C zur Reaktion gebracht. Nach Abkühlen auf Raumtemperatur wurden 5 ml Essigsäure zum Reaktionsgemisch getropft, wobei das gebildete 2-Mercapto-5-methylpyridin als gelbes Pulver ausfiel.

c) Zu einem Zweiphasengemisch aus 40 ml $CH_2Cl_2$, 20 ml 37%ige wässrige HCl und 3 g 2-Mercapto-5-methylpyridin auf -10°C gekühlt, tropfte man innerhalb von 30 Minuten 50 ml einer 1,2 molaren Natriumhypochlorit-Lösung. Anschliessend wurde die organische Phase dreimal mit $H_2O$ ausgeschüttelt. Nach Abdestillieren des Lösungsmittels erhielt man das 5-Methylpyridin-sulfochlorid als gelbliche Flüssigkeit.

d) Die Umsetzung des Sulfochlorids mit 25%iger $NH_4OH$-Lösung ergab das 5-Methylpyridin-2-sulfonamid.

e) 0,7 g 4,6-Dichlor-5-(2-methoxy-phenoxy)-2,2'-bipyrimidin, 520 mg 5-Methylpyridin-2-sulfonamid und 320 mg K-tert. Butylat in 2 ml DMSO gelöst wurden bei 80°C während 3 Stunden gerührt. Nach der üblichen Aufarbeitung des Reaktionsgemisches erhielt man 410 mg 5-Methyl-pyridin-2-sulfonsäure [6-chlor-5-(2-methoxy-phenoxy)-2,2'-bipyrimidin-4-yl]-amid.

Beispiel 32

In Analogie zu Beispiel 22 erhielt man aus 105 mg 5-Methyl-pyridin-2-sulfonsäure 6-(2-hydroxy-ethoxy)-5-(2-methoxy-phenoxy)-2,2'-bipyridmin-4-ylamid und 30 mg 2-Pyridyl-carbonsäureazid, 100 mg Pyridin-2-ylcarbaminsäure 2-[5-(2-methoxy-phenoxy)-6-(5-methyl-pyridin-2-ylsulfonylamino)-2,2'-bipyridimin-4-yloxy]-ethylester als beige Kristalle. Smp: Zersetzung bei 198°C.

Beispiel 33

a) In Analogie zu Beispiel 22 erhielt man aus 712 mg 4,6-Dichlor-5-(2-methoxy-phenoxy)-2-morpholin-4-yl-pyrimidin und (5-Methyl-pyridin-2-sulfonamid)-K 580 mg 5-Methyl-pyridin-2-sulfonsäure 6-Chlor-5-(2-methoxyphenoxy)-2-morpholin-4-yl-pyrimidin-4-ylamid.

b) Die Umsetzung dieser Verbindung mit Na-Glykolat ergab das 5-Methylpyridin-2-sulfonsäure 6-(2-hydroxy-ethoxy)-5-(2-methoxy-phenoxy)-2-morpholin-4-yl-pyrimidin-4-ylamid. Smp. 195-196°C (aus Ethanol).

Beispiel 34

In Analogie zu Beispiel 22 erhielt man 117 mg Pyridin-2-yl-carbaminsäure 2-[5-(2-methoxy-phenoxy)-6-(5-methyl-pyridin-2-ylsulfonylamino)-2-morpholin-4-yl-pyrimidin-4-yloxy]-ethylester aus 105 mg 5-Methyl-pyridin-2-sulfonsäure 6-(2-hydroxy-ethoxy)-5-(2-methoxy-phenoxy)-2-morpholin-4-yl-pyrimidin-4-ylamid und 2-Pyridyl-carbonsäureazid. Smp: Zersetzung bei 175°C.

Beispiel 35

105 mg 5-Methyl-pyridin-2-sulfonsäure 6-(2-hydroxy-ethoxy)-5-(2-methoxy-phenoxy)-2-morpholin-4-yl-pyrimidin-4-ylamid in 2 ml Dichlormethan wurde mit 3 ml einer 1,9 molaren Phosgen-Lösung in Toluol versetzt. Nach 1 Stunde bei Raumtemperatur war das Chloroformiat vollständig gebildet. Dann wurde der Ueberschuss an Reagens abdestilliert; der Rückstand wurde in ein Gemisch von Chloroform und Pyridin aufgenommen; 0,5 g 3-(Hydroxymethyl)-furan wurden zugegeben und man liess bei 60°C während 3 Stunden reagieren. Nach der üblichen Aufarbeitung wurde die Verbindung an Kieselgel gereinigt (Dichlormethan-Diethylether 4:1 in Vol. als Fliessmittel). Man erhielt 65 mg Carbonsäure Furan-3-yl-methylester 2-[5-(2-methoxy-phenoxy)-6-(5-methyl-pyridin-2-sulfonylamino)-2-morpholin-4-yl-pyrimidin-4-yloxy]-ethylester. MS: 640,5 $[M-H)^-]$.

Beispiel 36

9,2 g 4-[4-Chloro-5-(2-chloro-5-methoxy-phenoxy)-6-methyl-pyrimidin-2-yl]-morpholin und 17,8 g 5-Isopropyl-pyridin-2-sulfonamid-Kalium in 130 ml trockenem Dimethylsulfoxid wurden unter Argon 16 Stunden auf 120°C erwärmt. Danach wurde das Dimethylsulfoxid abdestilliert, der Rückstand zwischen Ethylacetat und 1N Salzsäure verteilt und die organische Phase neutral gewaschen. Die organische Phase wurde getrocknet, das Lösungsmittel abgedampft und der Rückstand aus Ethanol umkristallisiert. Man erhielt 10,3 g 5-Isopropyl-pyridin-2-sulfonsäure 5-(2-chloro-5-methoxyphenoxy)-6-methyl-2-morpholin-4-yl-pyrimidin-4-ylamid, MS: M = 534.

Beispiel 37

1 g der in Beispiel 36 erhaltenen Verbindung und 2,1 g Seleniumdioxid in 40 ml Dioxan wurden in einem Autoklaven bei 170°C während 7 Stunden gerührt. Das Reaktionsgemisch wurde filtriert und das Filtrat eingeengt. Der Rückstand wurde zwischen Essigester und Wasser verteilt. Die organische Phase wurde getrocknet, das Lösungsmittel abgedampft und der Rückstand über Kieselgel mit Essigester-Hexan gereinigt. Man erhielt 0,53 g 5-Isopropyl-pyridin-2-sulfonsäure 5-(2-chloro-5-methoxy-phenoxy)-6-formyl-2-morpholin-4-yl-pyrimidin-4-ylamid, Smp. 194°C.

Beispiel 38

0,1 g der in Beispiel 37 erhaltenen Verbindung in 3 ml Ethanol wurde mit 0,014 g Natriumborhydrid versetzt. Das Reaktionsgemisch wurde bei 80°C 1 Stunde gerührt. Danach wurde das Ethanol abdestilliert und der Rückstand zwischen Chloroform und 1N HCl verteilt. Die organische Phase wurde mit Wasser gewaschen und getrocknet, das Lösungsmittel abgedampft und der Rückstand mit Chloroform-Methanol über Kieselgel chromatographiert. Nach Umkristallisation aus Dichlormethan-Ethanol erhielt man 0,072 g 5-Isopropyl-pyridin-2-sulfonsäure 5-(2-chloro-5-methoxy-phenoxy)-6-hydroxymethyl-2-morpholin-4-yl-pyrimidin-4-ylamid. Smp. 105°C.

Beispiel 39

0,2 g der in Beispiel 38 erhaltenen Verbindung in 3,5 ml $POCl_3$ wurden mit 0,083 g $PCl_5$ während 2 Stunden bei 20°C gerührt. Danach wurde das $POCl_3$ abdestilliert und der Rückstand zwischen Essigester und wässrige Natriumbicarbon verteilt. Die organische Phase wurde mit Wasser gewaschen, getrocknet und das Lösungsmittel abgedampft. Der Rückstand wurde über Kieselgel mit Chloroform-Methanol chromatographiert, danach aus Dichlormethan-Ethanol umkristallisiert. Man erhielt 0,150 g 5-Isopropyl-pyridin-2-sulfonsäure 5-(2-chloro-5-methoxy-phenoxy)-6-chloromethyl-2-morpholin-4-yl-pyrimidin-4-ylamid. Smp. 205°C.

Beispiel 40

Zu einer Natriumglykolatlösung aus 0,35 g Ethylenglykol und 0,021 g Natrium wurden 0,130 g der in Beispiel 39 erhaltenen Verbindung gegeben. Das Reaktionsgemisch wurde unter Argon 2 Stunden bei 80°C gerührt. Danach wurde das Ethylenglykol abdestilliert und der Rückstand zwischen Ethylacetat und 1N Salzsäure verteilt. Die organische Phase wurde mit Wasser gewaschen, über Natriumsulfat getrocknet und das Lösungsmittel abdestilliert. Der Rückstand wurde in Ether-Petrolether umkristallisiert. Man erhielt 0,104 g 5-(2-Chloro-5-methoxy-phenoxy)-6-(2-hydroxy-ethoxymethyl)-2-morpholin-4-yl-pyrimidin-4-ylamid. Smp. 166°C.

Beispiel 41

In Analogie zu Beispiel 2 wurde aus der in Beispiel 40 erhaltenen Verbindung der Pyridin-2-ylcarbaminsäure 2-[5-(2-chloro-5-methoxyphenoxy)-6-(5-isopropyl-pyridin-2-sulfonylamino)-2-morpholin-4-yl-pyrimidin-4-yl-methoxy]-ethylester erhalten, MS: $(M-H)^-$ = 713.

Beispiel 42

In Analogie zu Beispiel 2 wurde aus der in Beispiel 38 erhaltenen Verbindung das Pyridin-2-ylcarbaminsäure 5-(2-chloro-5-methoxy-phenoxy)-6-(5-isopropyl-pyridin-2-ylsulfonylamino)-2-morpholin-4-yl-pyrimidin-4-yl-methylester erhalten, MS: $(M-H)^-$ = 669.

Beispiel 43

In Analogie zu Beispiel 40 wurde aus der in Beispiel 39 erhaltenen Verbindung und (RS)-2,2-Dimethyl-1,3-dioxolan-4-methanol-Na das 5-Isopropyl-pyridin-2-sulfonsäure (RS)-5-(2-chloro-5-methoxy-phenoxy)-6-(2,2-dimethyl-1,3-dioxolan-3-ylmethoxymethyl)-2-morpholin-4-yl-pyrimidin-4-ylamid erhalten, MS: $(M-H)^-$ = 663.

Beispiel 44

Eine Lösung von 0,05 g der in Beispiel 43 hergestellten Verbindung in 2 ml Dioxan wurde mit 2 ml 1N HCl versetzt und 15 Minuten auf 80°C erwärmt. Nach dem Eindampfen wurde der Rückstand über Kieselgel mit Chloroform-Methanol chromatographiert und lieferte das 5-Isopropylpyridin-2-sulfonsäure (RS)-5-(2-chloro-5-methoxy-phenoxy)-6-(2,3-dihydroxypropoxymethyl)-2-morpholin-4-yl-pyrimidin-4-ylamid. Smp. 116°C, MS: $(M-H)^-$ = 623.

Beispiel 45

345 mg Natrium wurden bei 80°C in 50 ml abs. Ethylenglykol gelöst. Man liess etwas abkühlen, und gab 1,56 g 5-Isopropyl-pyridin-2-sulfonsäure 6-chlor-5-(2-methoxy-phenoxy)-2-morpholin-4-yl-pyrimidin-4-ylamid zu. Die entstandene Lösung wurde während 24 Stunden bei 140°C gerührt, das Lösungsmittel am Hochvakuum entfernt und der Rückstand in 40 ml Wasser gelöst. Nach 4 Stunden bei 5°C wurde abgenutscht, die Kristalle in 40 ml Wasser suspendiert, mit Essigester überschichtet, und unter Rühren tropfenweise mit 1N wässriger HCl versetzt, bis das pH auf 3,5 gesunken war. Die wässrige Phase wurde dreimal mit Essigester extrahiert und die organischen Phasen zweimal mit Wasser und einmal mit gesättigter NaCl-Lösung gewaschen. Die vereinigten organischen Phasen wurden getrocknet und eingeengt, bis Kristallisation einsetzte (ca. 5 ml). Es wurde abgenutscht, mit Ether gewaschen und getrocknet. Man erhielt 1,144 g (70%) weisse Kristalle von 5-Isopropyl-pyridin-2-sulfonsäure 6-(2-hydroxy-ethoxy)-5-(2-methoxy-phenoxy)-2-morpholin-4-yl-pyrimidin-4-ylamid, Smp. 157-160°C, MS: $(M-H)^-$ = 544,4.

Herstellung der Ausgangsverbindung

Eine Lösung von 1,18 g 4,6-Dichlor-5-(2-methoxy-phenoxy)-2-morpholin-4-yl-pyrimidin und 2,12 g (8,88 mMol) 5-Isopropyl-pyridin-2-sulfonamid-Kaliumsalz in 25 ml getrocknetem DMSO wurden während 3 Stunden auf 80°C bis zum vollständigen Verschwinden des Dichlorids erwärmt. Das DMSO wurde am Hochvakuum entfernt, der Rückstand mit 60 ml Wasser aufgenommen, und die wässrige Lösung dreimal mit Diethylether gewaschen. Die Lösung wurde dann mit 1N HCl auf pH 3,5 angesäuert und das Produkt dreimal mit Essigester extrahiert. Die organischen Phasen wurden zweimal mit Wasser und schliesslich einmal mit gesättigter Kochsalz-Lösung gewaschen, vereinigt, mit Natriumsulfat getrocknet und eingedampft. Der kristalline Rückstand wurde zur vollständigen Entfernung einer Spur 5-Isopropyl-pyridin-2-sulfonamid zweimal mit absolutem Diethylether digeriert. Die zurückbleibenden Kristalle wurden abfiltriert und getrocknet. Man erhielt 1,66 g (96%) 5-Isopropyl-pyridin-2-sulfonsäure 6-chlor-5-(2-methoxy-phenoxy)-2-morpholin-4-yl-pyrimidin-4-ylamid als weisse Kristalle vom Smp. 168-176°C, MS: $(M-H)^-$ = 518,3.

Beispiel 46

In Analogie zu Beispiel 45 wurde nach 10 Stunden Reaktionszeit bei 120°C unter Zusatz von DMSO als Lösungsvermitler (Ethylenglykol:DMSO 5:2) das 5-tert.Butylthiophen-2-sulfonsäure 6-(2-hydroxy-ethoxy)-5-(2-methoxy-phenoxy-2-morpholin-4-yl-pyrimidin-4-ylamid als weisser fester Schaum in 54% Ausbeute, MS: 565,5 $(M+H)^+$ erhalten.

Beispiel 47

In Analogie zu Beispiel 45 wurde nach 3 Stunden Reaktionszeit bei 140°C 2,5-Dichlorthiophen-3-sulfonsäure 6-(2-hydroxy-ethoxy)-5-(2-methoxyphenoxy)-2-morpholin-4-yl-pyrimidin-4-ylamid als weisse Kristalle in 43% Ausbeute erhalten. Smp. 180-183°C. MS: 575,3 $(M-H)^-$.

Beispiel 48

In Analogie zu Beispiel 45 wurde nach 3,5 Stunden Reaktionszeit bei 140°C 3,5-Dimethylisoxazol-4-sulfonsäure 6-(2-hydroxy-ethoxy)-5-(2-methoxyphenoxy)-2-morpholin-4-yl-pyrimidin-4-ylamid, weisse Kristalle vom Smp. 144-147°C, MS: 520,4 $(M-H)^-$ erhalten.

Beispiel 49

110 mg Natrium wurden bei 50°C in 2,5 ml Ethylenglykol gelöst. Man liess auf Zimmertemperatur abkühlen und setzte 260 mg 2,5-Dichlorthiophen-3-sulfonsäure 6-chlor-5-(2-methoxy-phenoxy)-2,2'-bipyrimidin-4-ylamid zu. Nach Erwärmen auf 50°C während 2 Stunden wurde das Glykol am Hochvakuum entfernt und der feste Rückstand in 20 ml Wasser gelöst. Das Produkt wurde durch Zugabe von 0,3 ml Essigsäure ausgefällt. Nach Filtration, Waschen mit Wasser, und Trocknen am Hochvakuum bei 50°C wurden 182 mg (67%) hellbeige Kristalle von 2,5-Dichlorthiophen-3-sulfonsäure 6-(2-hydroxy-ethoxy)-5-(2-methoxy-phenoxy)-2,2'-bipyrimidin-4-ylamid, Smp. 157-160°C, MS: $M^+$ (569), 470 $(M^+-(SO_2+Cl))$ erhalten.

Herstellung der Ausgangsverbindung:

Eine Lösung von 0,349 g 4,6-Dichloro-5-(2-methoxy-phenoxy)-2,2'-bipyrimidin und 0,405 g 2,5-Dichlorthiophen-3-sulfonamid Kalium-Salz in 5 ml getrocknetem DMSO wurde während 16 Stunden bei Zimmertemperatur gehalten. Danach wurden 0,112 g K-tert.-butylat zugegeben, worauf die Reaktion innerhalb 5 Stunden beendet war. Das Reaktionsgemisch wurde auf 40 ml Eiswasser gegossen und zur Entfernung von überschüssigem Reagens mit 40 ml Diethylether extrahiert. Aus der wässrigen Phase wurde durch Aussalzen mit gesättiger Kochsalzlösung (20 ml) das 2,5-Dichlorthiophen-3-sulfonsäure 6-chlor-5-(2-methoxy-phenoxy)-2,2'-bipyrimidin-4-ylamid Natrium-Salz durch Filtration und Waschen mit Ether (0,54 g beiges Pulver) erhalten.

Zur Gewinnung des freien Sulfonamids wurde das Na-Salz in Wasser suspendiert, und die Suspension wurde mit Essigsäure angesäuert, und mit Essigester, dem ein wenig $CH_2Cl_2$ zugesetzt wurde, extrahiert. Die organische Phase wurde zweimal mit gesättigter Kochsalzlösung gewaschen, mit $MgSO_4$ getrocknet und unter vermindertem Druck eingedampft. Der Rückstand wurde mit Diethylether und Hexan kurz gewaschen und dann getrocknet. Man erhielt 0,30 g (54%) als beiges Pulver vom Smp. 140°C (Zers.).
MS: 444 $(M-(SO_2+Cl))$.

In Analogie wurde unter Verwendung von 3,5-Dimethylisoxazolyl-4-sulfonamid-Kalium das 3,5-Dimethylisoxazol-4-sulfonsäure 6-chlor-5-(2-methoxy-phenoxy)-2,2'-bipyrimidin-4-ylamid in 71% Ausbeute als beiges, leicht rötliches Pulver vom Smp. 184-187°C erhalten. MS: $M^+$ = 488, 393 $(M-(SO_2+OCH_3))$.

Beispiel 50

In Analogie zu Beispiel 49 wurde das 3,5-Dimethyl-isoxazol-4-sulfonsäure 6-(2-hydroxy-ethoxy)-5-(2-methoxy-phenoxy)-2,2'-bipyrimidin-4-ylamid als beiges Pulver vom Smp. 200-204°C. MS: 514 $(M^+)$, 450 $(M^+-SO_2)$, 419 $(450-CH_3O)$ erhalten.

Beispiel 51

Eine Lösung von 888 mg Pyridin-2-carbonylazid in 15 ml abs. Dioxan wurde 15 Minuten bei 80°C gehalten. Man liess etwas abkühlen, setzte 1,09 g der in Beispiel 45 hergestellten Verbindung zu und hielt die Lösung 4 Stunden bei 90°C. Danach dampfte man zur Trockene ein, nahm mit Essigester auf, wusch zweimal mit Wasser und einmal mit gesättigter Kochsalzlösung, vereinigte die organischen Phasen, trocknete und engte ein, wobei Kristalle des Produktes ausfiel. Zur endgültigen Reinigung chromatographierte man an Kieselgel mit $EtOAc/CH_2Cl_2$ (1:1), und erhielt 931 mg (70%) weisse Kristalle von Pyridin-2-ylcarbaminsäure 2-[6-(5-isopropylpyridin-2-ylsulfonylamino)-5-(2-methoxy-phenoxy)-2-morpholin-4-yl-pyrimidin-4-yloxy]ethylester vom Smp. 200-202°C. MS: 664,4 $(M-H)^-$. IR (KBr) 1730 $cm^{-1}$ (Carbamat).

Beispiel 52

In Analogie zu Beispiel 51 wurde aus der in Beispiel 49 hergestellten Verbindung in 61% Ausbeute der Pyridin-2-ylcarbaminsäure 2-[6-(2,5-dichlorthiophen-3-ylsulfonylamino)-5-(2-methoxy-phenoxy)-2,2'-bipyrimidin-4-yloxy] ethylester als weisse Kristalle vom Smp. 194-197°C, MS: 690,1 $(M+H)^+$, IR (KBr) 1732 $cm^{-1}$ (Carbamat) erhalten.

Beispiel 53

In Analogie zu Beispiel 51 wurde der aus der in Beispiel 50 hergestellten Verbindung in 68% Ausbeute Pyridin-2-ylcarbaminsäure 2-[5-(2-methoxy-phenoxy)-6-(3,5-dimethyl-isoxazol-4-ylsulfonylamino)-2,2'-bipyrimidin-4-yloxy] ethylester als hellgelbe Kristalle vom Smp. 217-218°C, MS: 635,3 $(M+H)^+$, IR (KBr) 1736 $cm^{-1}$ (Carbamat) erhalten.

Beispiel 54

In Analogie zu Beispiel 51 wurde der aus der in Beispiel 46 hergestellten Verbindung in 90% Ausbeute Pyridin-2-ylcarbaminsäure 2-[6-(5-tert-Butylthiophen-2-ylsulfonylamino)-5-(2-methoxy-phenoxy)-2-morpholin-4-yl-pyrimidin-4-yloxy]-ethylester, als weissen Schaum, MS: 683,5 $(M-H)^-$ erhalten.

Beispiel 55

In Analogie zu Beispiel 51 wurde aus der in Beispiel 47 hergestellten Verbindung in 55% Ausbeute der Pyridin-2-ylcarbaminsäure 2-[6-(2,5-dichloro-thiophen-3-ylsulfonylamino)-5-(2-methoxy-phenoxy)-2-morpholin-4-yl-pyrimidn-4-yloxy]-ethylester, weisse Kristalle vom Smp. 194-196°C, MS: 695,3 $(M-H)^-$ erhalten.

Beispiel 56

In Analogie zu Beispiel 51 wurde der aus der in Beispiel 48 hergestellten Verbindung in 70% Ausbeute Pyridin-2-ylcarbaminsäure 2-[6-(3,5-dimethyl-isoxazol-4-ylsulfonylamino)-5-(2-methoxy-phenoxy)-2-morpholin-4-yl-pyrimidin-4-yloxy]-ethylester, weisse Kristalle vom Smp. 106-109°C, MS: 640,4 $(M-H)^-$ erhalten.

Beispiel 57

Man löste 5-Isopropyl-pyridin-2-sulfonsäure 6-(2-hydroxy-ethoxy)-5-(2-methoxy-phenoxy)-2-morpholin-4-yl-pyrimidin-4-ylamid (54.5 mg) in N,N-Dimethylacetamid (5 ml), fügte bei Raumtemperatur 14.4 mg 60%ige NaH Suspension hinzu, rührte 20 Minuten bei Raumtemperatur nach und versetzte schliesslich mit 2-Chlorpyrimidin (11.7 mg). Die Reaktionsmischung wurde 18 Stunden bei Raumtemperatur gerührt, in Eiswasser gegossen, gesättigte $NH_4Cl$-Lösung zugegeben und mit Essigester extrahiert. Die organische Phase wurde mit Wasser gewaschen, über Magnesiumsulfat getrocknet und schliesslich am Rotationsverdampfer eingeengt. Der Rückstand wurde über Kieselgel mit Methylenchlorid/ Methanol (100/1) als Laufmittel chromatographiert. Man erhielt so 5-Isopropyl-pyridin-2-sulfonsäure {5-(2-methoxy-phenoxy)-2-morpholin-4-yl-6-[2-(pyrimidin-2-yloxy)-ethoxy]-pyrimidin-4-yl}-amid als weisse Kristalle.
MS: 624 (M+H)

Beispiel 58

a) In Analogie zu Beispiel 45 erhielt man durch Umsetzung von 5-Isopropylpyridin-2-sulfonsäure [6-chloro-2-(3-methoxy-benzyl)-5-(2-methoxy-phenoxy)pyrimidin-4-yl]-amid mit Na in Ethylenglykol das 5-Isopropyl-pyridin-2-sulfonsäure [6-(2-hydroxy-ethoxy)-2-(3-methoxy-benzyl)-5-(2-methoxyphenoxy)-pyrimidin-4-yl]-amid als weissen Schaum.
MS: 579.3 (M-H)

Herstellung der Ausgangsverbindungen:

b) Man löste 10,8 g 3-Methoxyphenylacetonitril in Ethanol (100 ml), und sättigte die Lösung mit Chlorwasserstoff bei Raumtemperatur. Anschliessend rührte man 12 Stunden bei Raumtemperatur, kühlte die Lösung auf 0° C und saugte die ausgefallenene Kristalle ab. Das Rohprodukt wurde aus Aceton/Diethylether umkristallisiert. Man erhielt so 2-(3-Methoxy-phenyl)-acetimidsäure-ethylester-hydrochlorid als weissen kristallinen Feststoff.

MS: 193 (M)

c) 2-(3-Methoxy-phenyl)-acetimidsäure-ethylester-hydrochlorid (12 g) wurden in Ethanol (100 ml) gelöst und bei -75° C mit 14 ml flüssigem Ammoniak versetzt. Man liess während 5 Stunden auf Raumtemperatur kommmen und engte am Rotationsverdampfer ein. Der Rückstand wurde in Aceton aufgeschlämmt, die ausgefallenen Kristalle abgesaugt und im Hochvakuum getrocknet. Man erhielt so das 2-(3-Methoxy-phenyl)-acetamidin-hydrochlorid als weissen, kristallinen Feststoff.
MS: 164 (M)

d) Man löste Na (2.3 g) in Methanol (40 ml) fügte bei Raumtemperatur nacheinander 2-(3-Methoxy-phenyl)-acetamidin-hydrochlorid (10 g) und (2-Methoxyphenoxy)malonsäure-dimethylester (12.67 g) zu und rührte 5 Stunden bei Raumtemperatur. Anschliessend wurde am Rotationsverdampfer eingeengt und das Rohprodukt auf Wasser gegeben. Die Wasserphase wurde mit Essigester gewaschen, anschliessend auf pH 1 gestellt und die dann ausfallenden Kristalle abgenutscht und im Hochvakuum getrocknet. Man erhielt so das 2-(3-Methoxy-benzyl)-5-(2-methoxy-phenoxy)-pyrimidin-4,6-diol als beige Kristalle.
MS: 354 (M)

e) Man löste 2-(3-Methoxy-benzyl)-5-(2-methoxy-phenoxy)-pyrimidin-4,6-diol (14 g) in Acetonitril (150 ml), versetzte bei Raumtemperatur mit Collidin (5.24 ml), Phosphoroxychlorid (21.7 ml) und rührte 9 Stunden bei Raumtemperatur. Man goss auf Eiswasser und extrahierte mit Essigester. Die organische Phase wurde mit halbgesättigter $KHCO_3$-Lösung gewaschen, über Magnesiumsulfat getrocknet und eingeengt. Der Rückstand wurde in Hexan/Diethylether aufgenommen, filtriert und das Filtrat am Rotationsverdampfer eingeengt. Man erhielt so das 4,6-Dichloro-2-(3-methoxy-benzyl)-5-(2-methoxy-phenoxy)-pyrimidin als hellbraune Kristalle.
MS: 390 (M)

f) In Analogie zu Beispiel 45 erhielt man durch Umsetzung von 4,6-Dichloro-2-(3-methoxy-benzyl)-5-(2-methoxy-phenoxy)-pyrimidin mit 5-Isopropyl-pyridin-2-sulfonamid-Kaliumsalz das 5-Isopropyl-pyridin-2-sulfonsäure [6-chloro-2-(3-methoxy-benzyl)-5-(2-methoxy-phenoxy)-pyrimidin-4-yl]-amid als gelben Schaum.
MS: 553.1 (M-H)

Beispiel 59

a) In Analogie zu Beispiel 45 erhielt man durch Umsetzung von 5-Isopropylpyridin-2-sulfonsäure [6-chloro-2-(3-methoxy-benzyl)-5-phenoxy-pyrimidin-4-yl]-amid mit Na in Ethylenglykol das 5-Isopropyl-pyridin-2-sulfonsäure [6-(2-hydroxy-ethoxy)-2-(3-methoxy-benzyl)-5-phenoxy-pyrimidin-4-yl]-amid als hellgelbe Kristalle.
MS: 549.2 (M-H)

Herstellung der Ausgangsverbindungen:

b) In Analogie zu Beispiel 58d erhielt man durch Kondensation von 2-(3-Methoxy-phenyl)-acetamidin hydrochlorid mit Phenoxymalonsäure-dimethylester das 2-(3-Methoxy-benzyl)-5-phenoxypyrimidin-4,6-diol als gelben Schaum.
MS: 324 (M)

c) In Analogie zu Beispiel 58e erhielt man durch Chlorierung von 2-(3-Methoxy-benzyl)-5-phenoxypyrimidin-4,6-diol mit Phosphoroxychlorid das 4,6-Dichloro-2-(3-methoxy-benzyl)-5-phenoxy-pyrimidin als gelben Kristalle.
MS: 360 (M)

d) In Analogie zu Beispiel 45 erhielt man durch Umsetzung von 4,6-Dichloro-2-(3-methoxy-benzyl)-5-phenoxy-pyrimidin mit 5-Isopropyl-pyridin-2-sulfonamid-Kaliumsalz das 5-Isopropyl-pyridin-2-sulfonsäure [6-chloro-2-(3-methoxy-benzyl)-5-phenoxy-pyrimidin-4-yl]-amid als gelben Schaum.
MS: 523 (M-H)

Beispiel 60

Man löste 5-Isopropyl-pyridin-2-sulfonsäure [6-(2-hydroxy-ethoxy)-2-(3-methoxy-benzyl)-5-phenoxy-pyrimidin-4-yl]-amid (55 mg) in trockenem Methylenchlorid (3 ml) und versetzte bei 0° C mit Bortribromid (50 mg ) in Methylenchlorid (2 ml). Man rührte 2 Stunden bei 0°C und weitere 4 Stunden bei Raumtemperatur, engte am Rotationverdampfer ein und chromatographierte den Rückstand über Kieselgel mit Methylenchlorid-Essigester als Laufmittel. Man erhielt so das 5-Isopropyl-pyridin-2-sulfonsäure [2-(3-hydroxy-benzyl)-6-(2-hydroxy-ethoxy)-5-phenoxy-pyrimidin-4-yl]-amid als weisse Kristalle.
MS: 525.1 (M-H)

Beispiel A

Tabletten enthaltend die folgenden Bestandteile können in herkömmlicher Weise hergestellt werden:

| Bestandteile | pro Tablette |
|---|---|
| Verbindung der Formel I | 10,0 - 100,0 mg |
| Lactose | 125,0 mg |
| Maisstärke | 75,0 mg |
| Talk | 4,0 mg |
| Magnesiumstearat | 1,0 mg |

Beispiel B

Kapseln enthaltend die folgenden Bestandteile können in herkömmlicher Weise hergestellt werden:

| Bestandteile | pro Kapsel |
|---|---|
| Verbindung der Formel I | 25,0 mg |
| Lactose | 150,0 mg |
| Maisstärke | 20,0 mg |
| Talk | 5,0 mg |

Beispiel C

Injektionslösungen können folgende Zusammensetzung aufweisen:

| | |
|---|---|
| Verbindung der Formel I | 3,0 mg |
| Gelatine | 150,0 mg |
| Phenol | 4,7 mg |
| Wasser für Injektionslösungen | ad 1,0 ml |

Beispiel D

In 3,5 ml Myglyol 812 und 0,08 g Benzylalkohol werden 500 mg Verbindung der Formel I suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

**Patentansprüche**

**1.** Verbindungen der Formel I,

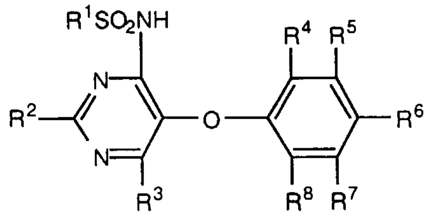

I

worin

$R^1$ ein mono- oder bicyclischer heterocyclischer Rest mit Sauerstoff, Stickstoff oder Schwefel als Heteroatom, ausgewählt aus 2-Furyl, 3-Furyl, Pyrimidinyl, 2-Pyridyl, 3-Pyridyl, 4-Pyridyl, 1,2-Diazinyl, 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl und Chinazolyl; oder ein obiger heterocyclischer Rest mono-oder di-substituiert mit $C_{1-7}$-alkyl, $C_{1-7}$-alkanoyl, Halogen, amino, mono-$C_{1-7}$-alkylamino, di-$C_{1-7}$-alkylamino oder mit einem weiteren obigen heterocyclischen Rest;

$R^2$ Wasserstoff, $C_{1-7}$-Alkyl, $C_{1-7}$-Alkoxy, $C_{1-7}$-Alkylthio, $C_{1-7}$-Alkoxy-$C_{1-7}$-alkyl, $C_{1-7}$-Alkylsulfonyl-$C_{1-7}$-alkoxy, Phenyl, $C_{1-7}$-Alkyl-phenyl, $C_{1-7}$-Alkoxy-phenyl, $C_{1-7}$-Alkylendioxyphenyl, Phenyl-$C_{1-7}$-alkyl, $C_{1-7}$-Alkyl-phenyl-$C_{1-7}$-alkyl, $C_{1-7}$-Alkoxy-phenyl-$C_{1-7}$-alkyl, $C_{1-7}$-Alkylendioxyphenyl-$C_{1-7}$-alkyl, oder ein mono- oder bicyclischer heterocyclischer Rest mit Sauerstoff, Stickstoff oder Schwefel als Heteroatom, ausgewählt aus 2-Furyl, 3-Furyl, Pyrimidinyl, 2-Pyridyl, 3-Pyridyl, 4-Pyridyl, 1,2-Diazinyl, 1,4-Diazinyl, Morpholino, 2-Thienyl, 3-Thienyl, Isoxazolyl, Oxazolyl, Thiazolyl, Imidazolyl, Pyrrolyl, Benzofuranyl, Benzothienyl, Indolyl, Purinyl, Thiomorpholino, Piperidino, Pyrrolidino, Benzodioxolyl, Chinolyl, Isochinolyl und Chinazolyl; oder ein obiger heterocyclischer Rest mono- oder di-substituiert mit $C_{1-7}$-alkyl, $C_{1-7}$-alkanoyl, Halogen oder mit einem weiteren obigen heterocyclischen Rest; oder Heterocyclyl-$C_{1-7}$-alkyl, wobei Heterocyclyl wie oben definiert ist;

$R^3$ $C_{1-7}$-Alkyl, $C_{1-7}$-Alkoxy, Formyl, Halogen-$C_{1-7}$-alkyl, Hydroxy-$C_{1-7}$-alkyl, Amino-$C_{1-7}$-alkyl oder einen Rest $-CH_2O-A-C_{1-7}$-alkyl, $-(CH_2)_m-O-(CR^aR^b)_nOH$, $-(CH_2)_m-O-(CR^3R^b)_nOR^9$, $-(CH_2)_m-O-(CR^aR^b)_nNH_2$ oder $-(CH_2)_m-O-(CR^aR^b)_n-B-R^9$;

$R^4$-$R^8$ Wasserstoff, $C_{1-7}$-Alkoxy oder Halogen;

$R^9$ ein mono- oder bicyclischer heterocyclischer Rest mit Sauerstoff, Stickstoff oder Schwefel als Heteroatom, ausgewählt aus 2-Furyl, 3-Furyl, Pyrimidinyl, 2-Pyridyl, 3-Pyridyl, 4-Pyridyl, 1,2-Diazinyl, 1,4-Diazinyl, Morpholino, 2-Thienyl, 3-Thienyl, Isoxazolyl, Oxazolyl, Thiazolyl, Imidazolyl, Pyrrolyl, Pyrazinyl, Benzofuranyl, Benzothienyl, Indolyl, Purinyl, Chinolyl, Isochinolyl und Chinazolyl; oder ein obiger heterocyclischer Rest mono- oder di-substituiert mit $C_{1-7}$-alkyl, $C_{1-7}$-alkanoyl, Halogen oder mit einem weiteren obigen heterocyclischen Rest; oder Phenyl oder durch $C_{1-7}$-Alkyl, $C_{1-7}$-Alkoxy und/oder Halogen substituiertes Phenyl, oder $C_{1-7}$-Alkyl;

$R^a$ und $R^b$ Wasserstoff oder $C_{1-7}$-Alkyl;

A eine ketalisierte 1,2-Dihydroxy-aethylengruppe,

B -OC(O)O-, -O(C(O)NH-, -NH(C(O)NH- oder -NHC(O)O-;

n 2,3 oder 4; und

m 0 oder 1

bedeuten.

**2.** Verbindungen gemäss Anspruch 1, worin $R^2$ Wasserstoff, $C_{1-7}$-Alkyl, $C_{1-7}$-Alkoxy, $C_{1-7}$-Alkylthio, $C_{1-7}$-Alkoxy-$C_{1-7}$-alkyl, $C_{1-7}$-Alkylsulfonyl-$C_{1-7}$-alkoxy, Phenyl, $C_{1-7}$-Alkoxy-phenyl, $C_{1-7}$-Alkylendioxyphenyl oder ein mono- oder bicyclischer heterocyclischer Rest mit Sauerstoff, Stickstoff oder Schwefel als Heteroatom, ausgewählt aus 2-Furyl, 3-Furyl, Pyrimidinyl, 2-Pyridyl, 3-Pyridyl, 4-Pyridyl, 1,2-Diazinyl, 1,4-Diazinyl, Morpholino, 2-Thienyl, 3-Thienyl,

Isoxazolyl, Oxazolyl, Thiazolyl, Imidazolyl, Pyrrolyl, Benzofuranyl, Benzothienyl, Indolyl, Purinyl, Thiomorpholino, Piperidino, Pyrrolidino, Benzodioxolyl, Chinolyl, Isochinolyl und Chinazolyl; oder ein obiger heterocyclischer Rest mono- oder di-substituiert mit $C_{1-7}$-alkyl, $C_{1-7}$-alkanoyl, Halogen oder mit einem weiteren obigen heterocyclischen Rest; oder Heterocyclyl-$C_{1-7}$-alkyl, wobei Heterocyclyl wie oben definiert ist;
$R^3$ $C_{1-7}$-Alkyl, $C_{1-7}$-Alkoxy, Formyl, Halogen-$C_{1-7}$-alkyl, Hydroxy-$C_{1-7}$-alkyl, Amino-$C_{1-7}$-alkyl oder einen Rest $-CH_2O-A-C_{1-7}$-alkyl, $-(CH_2)_m-O-(CR^aR^b)_nOH$, $-(CH_2)_m-O-(CR^aR^b)_nNH_2$ oder $-(CH_2)_m-O-(CR^aR^b)_n-B-R^9$ ist, und $R^1$, $R^4$-$R^9$, $R^a$, $R^b$, A, B, n und m die in Anspruch 1 angegebene Bedeutung haben.

**3.** Verbindungen gemäss Anspruch 1 oder 2, worin $R^1$ unsubstituiertes oder durch $C_{1-7}$-Alkyl, Halogen, Amino, mono- oder di-$C_{1-7}$-Alkylamino oder $C_{1-7}$-Alkanoyl substituiertes Pyridyl, Pyrimidinyl, Isoxazolyl, Furyl oder Thienyl ist.

**4.** Verbindungen gemäss einem der Ansprüche 1 -3, worin $R^2$ Wasserstoff, Pyrimidinyl, Pyridyl, Morpholino, Thiomorpholino, Piperidino, Pyrrolidino, Benzodioxolyl, $C_{1-7}$-Alkoxyphenyl oder $C_{1-7}$-Alkylthio ist.

**5.** Verbindungen gemäss einem der Ansprüche 1 -4, worin $R^3$ ein Rest $-O-(CR^aR^b)_nOH$, $-O-(CR^aR^b)_nNH_2$ oder ein Rest $-O(CH_2)_2-B-R^9$, und $R^9$ Pyridyl, Pyrimidinyl oder Furyl ist.

**6.** Verbindungen gemäss Anspruch 5, worin $R^3$ ein Rest $-O(CH_2)_2-B-R^9$ und B - (O)C(O)NH- ist.

**7.** Verbindungen gemäss Anspruch 6, worin $R^9$ 2-Pyridyl ist.

**8.** Die Verbindung gemäss Anspruch 7,
Pyridin-2-ylcarbaminsäure 2-[6-(5-isopropyl-pyridin-2-ylsulfonylamino)-5-(2-methoxy-phenoxy)-2-morpholin-4-yl-pyrimidin-4-yloxy]ethylester.

**9.** Die Verbindungen gemäss Anspruch 7,

Pyridin-2-ylcarbaminsäure 2-[6-(5-tert-butyl-thiophen-2-ylsulfonylamino)-5-(2-methoxy-phenoxy)-2,2'-bipyrimidin-4-yloxy]-ethylester,
Pyridin-2-ylcarbaminsäure 2-[5-(2-methoxy-phenoxy)-6-(5-pentyl-thiophen-2-ylsulfonylamino)-2,2'-bipyrimidin-4-yloxy]-ethylester,
Pyridin-2-ylcarbaminsäure 2-[6-[5-(2,2-dimethylpropionyl)-thiophen-2-ylsulfonylamino]-5-(2-methoxy-phenoxy)-2,2'-bipyrimidin-4-yloxy]-ethylester,
Pyridin-2-ylcarbaminsäure 2-[6-(5-isopropyl-pyridin-2-ylsulfonylamino)-5-(2-methoxy-phenoxy)-2,2'-bipyrimidin-4-yloxy]-ethylester,
Pyridin-2-ylcarbaminsäure 2-[5-(2-methoxy-phenoxy)-6-pyridin-2-ylsulfonylamino)-2,2'-bipyrimidin-4-yloxy]-ethylester,
Pyridin-2-ylcarbaminsäure 2-[5-(2-methoxy-phenoxy)-6-pyridin-3-ylsulfonylamino)-2,2'-bipyrimidin-4-yloxy]-ethylester,
Pyridin-2-ylcarbaminsäure 2-[6-(5-tert-butyl-thiophen-2-ylsulfonylamino)-5-(2-chloro-5-methoxy-phenoxy)-pyrimidin-4-yloxy]-ethylester,
Pyridin-2-yl-carbaminsäure 2-[6-(5-isopropyl-pyridin-2-ylsulfonylamino)-5-(2-methoxy-phenoxy)-2-(3-methoxy-phenyl)-pyrimidin-4-yloxy]-ethylester,
Pyridin-2-ylcarbaminsäure 2-[6-(5-isopropyl-pyridin-2-ylsulfonylamino)-5-(2-methoxy-phenoxy)-2-methylsulfanyl-pyrimidin-4-yloxy]-ethylester,
Pyridin-2-ylcarbaminsäure 2-[2-(1,3-benzodioxol-5-yl)-6-(5-isopropyl-pyridin-2-ylsulfonylamino)-5-(2-methoxy-phenoxy)-pyrimidin-4-yloxy]-ethylester,
Pyridin-2-ylcarbaminsäure 2-[5-(2-chlor-5-methoxy-phenoxy)-6-(5-isopropylpyridin-2-ylsulfonylamino)-2-morpholin-4-ylpyrimidin-4-yloxy]-ethylester,
Pyridin-2-ylcarbaminsäure 2-[5-(2-methoxy-phenoxy)-6-(5-methyl-pyridin-2-ylsulfonylamino)-2,2'-bipyridimin-4-yloxy]-ethylester,
Pyridin-2-yl-carbaminsäure 2-[5-(2-methoxy-phenoxy)-6-(5-methyl-pyridin-2-ylsulfonylamino)-2-morpholin-4-yl-pyrimidin-4-yloxy]-ethylester,
Pyridin-2-ylcarbaminsäure 2-[5-(2-chloro-5-methoxy-phenoxy)-6-(5-isopropylpyridin-2-sulfonylamino)-2-morpholin-4-yl-pyrimidin-4-yl-methoxy]-ethylester,
Pyridin-2-ylcarbaminsäure 5-(2-chloro-5-methoxy-phenoxy)-6-(5-isopropyl-pyridin-2-ylsulfonylamino)-2-morpholin-4-yl-pyrimidin-4-ylmethylester,
Pyridin-2-ylcarbaminsäure 2-[6-(2,5-dichlorthiophen-3-ylsulfonylamino)-5-(2-methoxy-phenoxy)-2,2'-bipyrimidin-4-yloxy]ethylester,
Pyridin-2-ylcarbaminsäure 2-[5-(2-methoxy-phenoxy)-6-(3,5-dimethyl-isoxazol-4-ylsulfonylamino)-2,2'-bipyrimidin-4-yloxy]ethylester,
Pyridin-2-ylcarbaminsäure 2-[6-(5-tert-Butylthiophen-2-ylsulfonylamino)-5-(2-methoxy-phenoxy)-2-morpholin-4-yl-pyrimidin-4-yloxy]-ethylester,
Pyridin-2-ylcarbaminsäure 2-[6-(2,5-dichloro-thiophen-3-ylsulfonylamino)-5-(2-methoxy-phenoxy)-2-morpholin-4-yl-pyrimidn-4-yloxy]-ethylester,
Pyridin-2-ylcarbaminsäure 2-[6-(3,5-dimethyl-isoxazol-4-ylsulfonylamino)-5-(2-methoxy-phenoxy)-2-morpholin-4-yl-pyrimidin-4-yloxy]-ethylester.

**10.** Verbindungen gemäss Anspruch 6, worin $R^9$ 4-Pyridyl ist.

**11.** Die Verbindung gemäss Anspruch 10,
Pyridin-4-ylcarbaminsäure 2-[6-(5-tert-butyl-thiophen-2-ylsulfonylamino)-5-(2-chloro-5-methoxy-phenoxy)-pyrimidin-4-yloxy]-ethylester.

**12.** Verbindungen gemäss Anspruch 5, worin $R^3$ ein Rest $-O(CH_2)_2$-B-$R^9$ und B -(O)C(O)O- ist.

**13.** Die Verbindungen gemäss Anspruch 12,

Carbonsäure Furan-3-ylmethylester 2-[5-(2-methoxy-phenoxy)-6-(5-methyl-pyridin-2-sulfonylamino)-2-morpholin-4-yl-pyrimidin-4-yloxy]-ethylester,
5-Isopropyl-pyridin-2-sulfonsäure {5-(2-methoxy-phenoxy)-2-morpholin-4-yl-6-[2-(pyrimidin-2-yloxy)-ethoxy]-pyrimidin-4-yl}-amid.

**14.** Verbindungen gemäss Anspruch 5, worin $R^3$ ein Rest $-O(CH_2)_2$-B-$R^9$ und B - NHC(O)NH- ist.

**15.** Die Verbindungen gemäss Anspruch 14,

5-tert-Butyl-thiophen-2-sulfonsäure 5-(2-methoxy-phenoxy)-6-[2-(3-pyridin-2-yl-ureido)-ethoxy]-2,2'-bipyrimidin-4-ylamid,
5-Isopropyl-pyridin-2-sulfonsäure 5-(2-methoxy-phenoxy)-6-[2-(3-pyridin-2-yl-ureido)-ethoxy]-2,2'-bipyrimidin-4-ylamid .

**16.** Verbindungen gemäss den Ansprüchen 1-4, worin $R^3$ Hydroxyäthoxy ist.

**17.** Die Verbindungen gemäss Anspruch 16,

5-tert-Butyl-thiophen-2-sulfonsäure 6-(2-hydroxy-ethoxy)-5-(2-methoxy-phenoxy)-2,2'-bipyrimidin-4-ylamid,
5-Pentyl-thiophen-2-sulfonsäure 6-(2-hydroxy-ethoxy)-5-(2-methoxy-phenoxy)-2,2'-bipyrimidin-4-ylamid,
5-(2,2-Dimethyl-propionyl)-thiophen-2-sulfonsäure 6-(2-hydroxy-ethoxy)-5-(2-methoxy-phenoxy)-2,2'-bipyrimidin-4-ylamid,
5-Isopropyl-pyridin-2-sulfonsäure 6-(2-hydroxy-ethoxy)-5-(2-methoxy-phenoxy)-2,2'-bipyrimidin-4-ylamid,
Pyridin-3-sulfonsäure 6-(2-hydroxy-ethoxy)-5-(2-methoxy-phenoxy)-2,2'-bipyrimidin-4-ylamid,
5-tert-Butyl-thiophen-2-sulfonsäure 5-(2-chloro-5--methoxy-phenoxy)-6-(2-hydroxyethoxy)-pyrimidin-4-ylamid,
5-tert-Butyl-thiophen-2-sulfonsäure 6-(2-hydroxy-ethoxy)-5-(2-methoxy-phenoxy)-pyrimidin-4-ylamid,
N-[5-(2-Chlor-5-methoxy-phenoxy)-6-(2-hydroxy-ethoxy)-pyri midin-4-yl)-5-isopropyl-pyridin-2-sulfonamid,
5-Isopropyl-N-[6-(2-hydroxy-ethoxy)-5-(2-methoxy-phenoxy)-2-(3-methoxy-phenyl)-pyrimidin-4-yl]-pyridin-2-sulfonamid,
5-Isopropyl-N-[6-(2-hydroxy-ethoxy)-5-(2-methoxy-phenoxy)-2-methylsulfanyl-pyrimidin-4-yl]-pyridin-2-sulfonamid,
N-[2-(1,3-Benzodioxol-5-yl)-6-(2-hydroxy-ethoxy)-5-(2-methoxy-phenoxy)-pyrimidin-4-yl)-5-isopropyl-pyridin-2-sulfonamid,
N-[5-(2-Chlor-5-methoxy-phenoxy)-6-(2-hydroxy-ethoxy)-2-morpholin-4-yl-pyrimidin-4-yl]-5-isopropyl-pyridin-2-sulfonamid,
5-Methyl-pyridin-2-sulfonsäure 6-(2-hydroxy-ethoxy)-5-(2-methoxy-phenoxy)-2,2'-bipyrimidin-4-ylamid,
5-Methyl-pyridin-2-sulfonsäure 6-(2-hydroxy-ethoxy)-5-(2-methoxy-phenoxy)-2-morpholin-4-yl-pyrimidin-4-ylamid,
5-Isopropyl-pyridin-2-sulfonsäure 6-(2-hydroxy-ethoxy)-5-(2-methoxy-phenoxy)-2-morpholin-4-yl-pyrimidin-4-ylamid,
5-tert.Butylthiophen-2-sulfonsäure 6-(2-hydroxy-ethoxy)-5-(2-methoxy-phenoxy-2-morpholin-4-yl-pyrimidin-4-ylamid,
2,5-Dichlorthiophen-3-sulfonsäure 6-(2-hydroxy-ethoxy)-5-(2-methoxy-phenoxy)-2-morpholin-4-yl-pyrimidin-4-ylamid,
3,5-Dimethylisoxazol-4-sulfonsäure 6-(2-hydroxy-ethoxy)-5-(2-methoxy-phenoxy)-2-morpholin-4-yl-pyrimidin-4-ylamid,
2,5-Dichlorthiophen-3-sulfonsäure 6-(2-hydroxy-ethoxy)-5-(2-methoxy-phenoxy)-2,2'-bipyrimidin-4-ylamid,
3,5-Dimethyl-isoxazol-4-sulfonsäure 6-(2-hydroxy-ethoxy)-5-(2-methoxy-phenoxy)-2,2'-bipyrimidin-4-ylamid,
5-Isopropyl-pyridin-2-sulfonsäure [6-(2-hydroxy-ethoxy)-2-(3-methoxy-benzyl)-5-(2-methoxy-phenoxy)-pyrimidin-4-yl]-amid,
5-Isopropyl-pyridin-2-sulfonsäure [6-(2-hydroxy-ethoxy)-2-(3-methoxy-benzyl)-5-phenoxy-pyrimidin-4-yl]-amid,
5-Isopropyl-pyridin-2-sulfonsäure [2-(3-hydroxy-benzyl)-6-(2-hydroxy-ethoxy)-5-phenoxy-pyrimidin-4-yl]-amid.

**18.** Verbindungen gemäss den Ansprüchen 1-4, worin $R^3$ Aminoethoxy ist.

**19.** Die Verbindungen gemäss Anspruch 18,
5-tert-Butyl-thiophen-2-sulfonsäure 6-(2-amino-ethoxy)-5-(2-methoxy-phenoxy)-2,2'-bipyrimidin-4-ylamid.

**20.** Verbindungen gemäss den Ansprüchen 1-4, worin $R^3$ $C_{1-7}$-Alkyl, $C_{1-7}$-Alkoxy, Formyl, Halogen-$C_{1-7}$-alkyl, Hydroxy-$C_{1-7}$-alkyl, oder $-CH_2O-A-C_{1-7}$-alkyl ist.

**21.** Die Verbindungen gemäss Anspruch 20,

5-Isopropyl-pyridin-2-sulfonsäure 5-(2-chloro-5-methoxy-phenoxy)-6-methyl-2-morpholin-4-yl-pyrimidin-4-ylamid,
5-(2-chloro-5-methoxy-phenoxy)-6-formyl-2-morpholin-4-yl-pyrimidin-4-ylamid,
5-Isopropyl-pyridin-2-sulfonsäure 5-(2-chloro-5-methoxy-phenoxy)-6-hydroxymethyl-2-morpholin-4-yl-pyrimidin-4-ylamid,
5-Isopropyl-pyridin-2-sulfonsäure 5-(2-chloro-5-methoxy-phenoxy)-6-chloromethyl-2-morpholin-4-yl-pyrimidin-4-ylamid,
5-(2-Chloro-5-methoxy-phenoxy)-6-(2-hydroxy-ethoxymethyl)-2-morpholin-4-yl-pyrimidin-4-ylamid.

**22.** Verbindungen der Formel

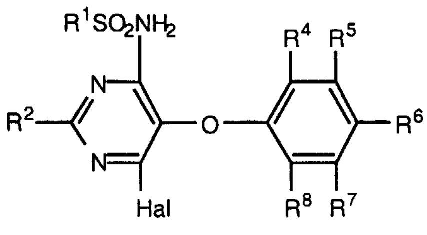

II

worin

$R^1$, $R^2$ und $R^4$- $R^8$ die in Anspruch 1 genannte Bedeutung haben und
Hal Halogen ist.

**23.** Die Verbindungen der Ansprüche 1-21 zur Anwendung als Heilmittel, insbesondere zur Behandlung von Erkrankungen, die mit Endothelin-Aktivitäten assoziiert sind, insbesondere Kreislauferkrankungen wie Hypertonie, Ischämie, Vasopasmen und Angina pectoris.

**24.** Verfahren zur Herstellung von Verbindungen der Ansprüche 1-21, dadurch gekennzeichnet, dass man

a) eine Verbindung der Formel

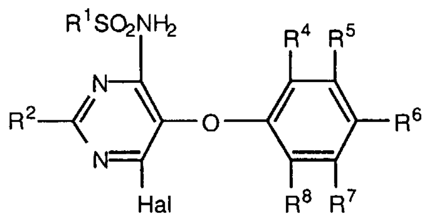

II

worin

$R^1$, $R^2$ und $R^4$ - $R^8$ die oben genannte Bedeutung haben und Hal Halogen ist,

mit einer Verbindung der Formel

$$HO(CR^aR^b)_nXH$$

worin n, $R^a$, und $R^b$ die oben genannte Bedeutung haben und X O oder NH darstellt, umsetzt, oder

b) eine Verbindung der Formel

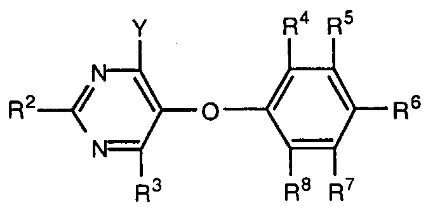

III

worin $R^2$ - $R^8$ die oben genannte Bedeutung haben, mit einer Verbindung der Formel

$$R^1SO_2Z$$

worin $R^1$ die oben genannte Bedeutung hat und wobei Y Halogen und Z Amino, oder Y Amino und Z Halogen darstellen, umsetzt, oder

c) eine Verbindung der Formel

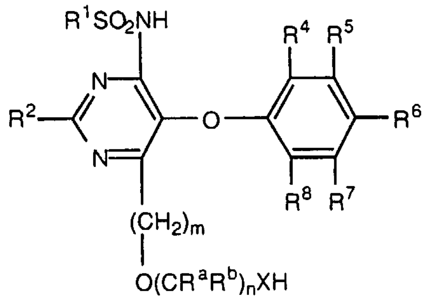

IV

worin $R^1$,$R^2$, $R^4$ - $R^8$, $R^a$, $R^b$, X, m und n die oben genannte Bedeutung haben,

c1) mit einem Isocyanat der Formel $R^9NCO$ oder einem Carbamoylchlorid der Formel $R^9NCOCl$ umsetzt, worin $R^9$ die oben genannte Bedeutung hat, oder

c2) mit Phosgen und danach mit einem Alkohol der Formel $R^9OH$; oder mit einem Chlorameisensäureester der Formel $R^9OC(O)Cl$ umsetzt; oder

d) eine Verbindung der Formel I, in der $R^3$ Halogen-$C_{1-7}$-alkyl darstellt, mit einer Verbindung der Formel

$$HOCH_2\text{-A-}C_{1-7}\text{-alkyl}$$

worin A eine ketalisierte 1,2-Dihydroxy-aethylengruppe darstellt,
umsetzt,

und gegebenenfalls in der erhaltenen Verbindung der Formel I enthaltene Substituenten abwandelt und/oder die erhaltene Verbindung der Formel I in ein Salz überführt.

**25.** Pharmazeutische Präparate, enthaltend eine Verbindung der Ansprüche 1-21 und übliche Träger- und Hilfsstoffe.

**26.** Verbindungen der Ansprüche 1-21, wenn hergestellt nach dem Verfahren von Anspruch 23.

**27.** Verwendung von Verbindungen der Ansprüche 1-21 als Wirkstoffe bei der Herstellung von Heilmitteln zur Behandlung von Erkrankungen, die mit Endothelin-Aktivitäten assoziiert sind, insbesondere Kreislauferkrankungen wie Hypertonie, Ischämie, Vasopasmen und Angina pectoris.

**28.** Verbindungen gemäß Ansprüche 1-21 zur Anwendung in einem Verfahren zur Behandlung von Erkrankungen, die mit Endothelin-Aktivitäten assoziiert sind, insbesondere Kreislauferkrankungen wie Hypertonie, Ischämie, Vasopasmen und Angina pectoris durch Verabreichung einer wirksamen Menge einer Verbindung der Ansprüch 1-21 an eine einer solchen Behandlung bedürftigen Person.

## Claims

**1.** Compounds of formula I,

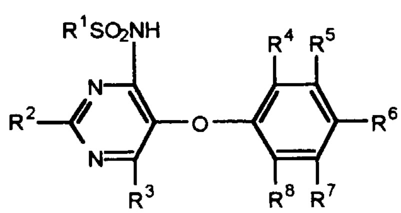

I

wherein

$R^1$ signifies a mono- or bicyclic heterocyclic residue with oxygen, nitrogen or sulphur as the hetero atom, selected from 2-furyl, 3-furyl, pyrimidinyl, 2-pyridyl, 3-pyridyl, 4-pyridyl, 1,2-diazinyl, 1,4-diazinyl, morpholino, 2-thienyl, 3-thienyl, isoxazolyl, oxazolyl, thiazolyl, imidazolyl, pyrrolyl, benzofuranyl, benzothienyl, indolyl, purinyl, quinolyl, isoquinolyl and quinazolyl; or an above heterocyclic residue mono- or di-substituted with $C_{1-7}$-alkyl, $C_{1-7}$-alkanoyl, halogen, amino, mono-$C_{1-7}$-alkylamino, di-$C_{1-7}$-alkylamino or with another of the above heterocyclic residues;

$R^2$ signifies hydrogen, $C_{1-7}$-alkyl, $C_{1-7}$-alkoxy, $C_{1-7}$-alkylthio, $C_{1-7}$-alkoxy- $C_{1-7}$-alkyl, $C_{1-7}$-alkylsulphonyl-$C_{1-7}$-alkoxy, phenyl, $C_{1-7}$-alkyl-phenyl, $C_{1-7}$-alkoxy-phenyl, $C_{1-7}$-alkylenedioxyphenyl, phenyl- $C_{1-7}$-alkyl, $C_{1-7}$-alkyl-phenyl- $C_{1-7}$-alkyl, $C_{1-7}$-alknxyl-phenyl-$C_{1-7}$-alkyl, $C_{1-7}$-alkylenedioxy-phenyl- $C_{1-7}$-alkyl, or a mono- or bicyclic heterocyclic residue with oxygen, nitrogen or sulphur as the hetero atom, selected from 2-furyl, 3-furyl, pyrimidinyl, 2-pyridyl, 3-pyridyl, 4-pyridyl, 1,2-diazinyl, 1,4-diazinyl, morpholino, 2-thienyl, 3-thienyl, isoxazolyl, oxazolyl, thiazolyl, imidazolyl, pyrrolyl, benzofuranyl, benzothienyl, indolyl, purinyl, thiomorpholino, piperidino, pyrrolidino, benzodioxolyl, quinolyl, isoquinolyl and quinazolyl; or an above heterocyclic residue mono- or di-substituted with $C_{1-7}$-alkyl, $C_{1-7}$-alkanoyl, halogen or with another of the above heterocyclic residues; or heterocyclyl-$C_{1-7}$-alkyl in which heterocyclyl is as defined above;

$R^3$ signifies $C_{1-7}$-alkyl, $C_{1-7}$-alkoxy, formyl, halo-$C_{1-7}$-alkyl, hydroxy-$C_{1-7}$-alkyl, amino$C_{1-7}$-alkyl or a residue $-CH_2O-A-C_{1-7}$-alkyl, $-(CH_2)_m-O-(CR^aR^b)_nOH$, $-(CH_2)_m-O-(CR^aR^b)_nOR^9$, $-(CH_2)_m-O-(CR^aR^b)_nNH_2$ or $-(CH_2)_m-O-(CR^aR^b)_n-B-R^9$;

$R^4$-$R^8$ signify hydrogen, $C_{1-7}$-alkoxy or halogen;

$R^9$ signifies a mono- or bicyclic heterocyclic residue with oxygen, nitrogen or sulphur as the hetero atom, selected from 2-furyl, 3-furyl, pyrimidinyl, 2-pyridyl, 3-pyridyl, 4-pyridyl, 1,2-diazinyl, 1,4-diazinyl, morpholino, 2-thienyl, 3-thienyl, isoxazolyl, oxazolyl, thiazolyl, imidazolyl, pyrrolyl, pyrazinyl, benzofuranyl, benzothienyl, indolyl, purinyl, quinolyl, isoquinolyl and quinazolyl; or an above heterocyclic residue mono- or di-substituted with $C_{1-7}$-alkyl, $C_{1-7}$-alkanoyl, halogen or with another of the above heterocyclic residues; or phenyl or phenyl substituted by $C_{1-7}$-alkyl, $C_{1-7}$-alkoxy and/or halogen, or $C_{1-7}$-alkyl;

$R^a$ and $R^b$ signify hydrogen or $C_{1-7}$-alkyl;

A signifies a ketalized 1,2-dihydroxy-ethylene group;

B signifies -OC(O)O-, -O(C(O)NH-, -NH(C(O)NH- or -NHC(O)O-:

n signifies 2, 3 or 4; and

m signifies 0 or 1.

**2.** Compounds according to claim 1, wherein $R^2$ is hydrogen, $C_{1-7}$-alkyl, $C_{1-7}$-alkoxy, $C_{1-7}$-alkylthio, $C_{1-7}$-alkoxy- $C_{1-7}$-alkyl, $C_{1-7}$-alkylsulphonyl-$C_{1-7}$-alkoxy, phenyl, $C_{1-7}$-alkoxy-phenyl, $C_{1-7}$-alkylenedioxyphenyl or a mono- or bicyclic heterocyclic residue with oxygen, nitrogen or sulphur as the hetero atom, selected from 2-furyl, 3-furyl, pyrimidinyl, 2-pyridyl, 3-pyridyl, 4-pyridyl, 1,2-diazinyl, 1,4-diazinyl, morpholino, 2-thienyl, 3-thienyl, isoxazolyl, oxazolyl, thiazolyl, imidazolyl, pyrrolyl, benzofuranyl, henzothienyl, indolyl, purinyl, thiomorpholino, piperidino, pyrrolidino, benzodioxolyl, quinolyl, isoquinolyl and quinazolyl; or an above heterocyclic residue mono- or di-substituted with $C_{1-7}$-alkyl, $C_{1-7}$-alkanoyl, halogen or with another of the above heterocyclic residues; or heterocyclyl-$C_{1-7}$-alkyl in which heterocyclyl is as defined above;
$R^3$ is $C_{1-7}$-alkyl, $C_{1-7}$-alkoxy, formyl, halo-$C_{1-7}$-alkyl, hydroxy-$C_{1-7}$-alkyl, amino-$C_{1-7}$-alkyl or a residue $CH_2-A-C_{1-7}$-alkyl, $-(CH_2)_m-O-(CR^aR^b)_nOH$, $-(CH_2)_m-O-(CR^aR^b)_nNH_2$ or $-(CH_2)_m-O-(CR^aR^b)_n-B-R^9$ and $R^1$, $-R^9$, $R^a$, $R^b$, A, B, n and m have the significance given in claim 1.

**3.** Compounds according to claim 1 or 2, wherein $R^1$ is pyridyl, pyrimidinyl, isoxazolyl, furyl or thienyl which is unsubstituted or substituted by $C_{1-7}$-alkyl, halogen, amino, mono- or di-$C_{1-7}$-alkylamino or $C_{1-7}$-alkanoyl.

**4.** Compounds according to any one of claims 1-3, wherein $R^2$ is hydrogen, pyrimidinyl, pyridyl, morpholino, thiomor-

pholino, piperidino, pyrrolidino, benzodioxolyl, $C_{1-7}$-alkoxyphenyl or $C_{1-7}$-alkylthio.

5. Compounds according to any one of claims 1 -4, wherein $R^3$ is a residue $-O-(CR^aR^b)_nOH$, $-O-(CR^aR^b)_nNH_2$ or a residue $-O(CH_2)_2-B-R^9$ and $R^9$ is pyridyl, pyrimidinyl or furyl.

6. Compounds according to claim 5, wherein $R^3$ is a residue $-O(CH_2)_2-B-R^9$ and B is -(O)C(O)NH-.

7. Compounds according to claim 6, wherein $R^9$ is 2-pyridyl.

8. The compound according to claim 7,
pyridin-2-ylcarbamic acid 2-[6-(5-isopropyl-pyridin-2-ylsulphonylamino)-5-(2-methoxy-phenoxy)-2-morpholin-4-ylpyrimidin-4-yloxy]ethyl ester.

9. The compounds according to claim 7,

pyridin-2-ylcarbamic acid 2-[6-(5-tert-butyl-thiophen-2-ylsulphonylamino)-5-(2-methoxy-phenoxy)-2,2'-bipyrimidin-4-yloxy] -ethyl ester,
pyridin-2-ylcarbamic acid 2-[5-(2-methoxy-phenoxy)-6-(5-pentyl-thiophen-2-yl-sulphonylamino)-2,2'-bipyrimidin-4-yloxy] -ethyl ester,
pyridin-2-ylcarbamic acid 2-[6-[5-(2,2-dimethylpropionyl)-thiophen-2-yl-sulphonylamino] -5-(2-methoxy-phenoxy)-2,2'-bipyrimidin-4-yloxy]-ethyl ester,
pyridin-2-ylcarbamic acid 2-[6-(5-isopropyl-pyridin-2-ylsulphonylamino)-5-(2-methoxy-phenoxy)-2,2'-bipyrimidin-4-yloxy]-ethyl ester,
pyridin-2-ylcarbamic acid 2-(5-(2-methoxy-phenoxy)-6-pyridin-2-ylsulphonylamino)-2,2'-bipyrimidin-4-yloxy]-ethyl ester,
pyridin-2-ylcarbamic acid 2-[5-(2-methoxy-phenoxy)-6-pyridin-3-ylsulphonylamino)-2,2'-bipyrimidin-4-yloxy]-ethyl ester,
pyridin-2-ylcarbamic acid 2-(6-(5-tert-butyl-thiophen-2-ylsulphonylamino)-5-(2-chloro-5-methoxy-phenoxy)-pyrimidin-4-yloxy]-ethyl ester,
pyridin-2-yl-carbamic acid 2-[6-(5-isopropyl-pyridin-2-ylsulphonylamino)-5-(2-methoxy-phenoxy)-2-(3-methoxy-phenyl)-pyrimidin-4-yloxy]-ethyl ester,
pyridin-2-ylcarbamic acid 2-(6-(5-isopropyl-pyridin-2-ylsulphonylamino)-5-(2-methoxy-phenoxy)-2-methylsulphanyl-pyrimidin-4-yloxy] -ethyl ester,
pyridin-2-ylcarbamic acid 2-[2-(1,3-benzodioxol-5-yl)-6-(5-isopropyl-pyridin-2-ylsulphonylamino)-5-(2-methoxy-phenoxy)-pyrimidin-4-yloxy]-ethyl ester,
pyridin-2-ylcarbamic acid 2-[5-(2-chloro-5-methoxy-phenoxy)-6-(5-isopropylpyridin-2-ylsulphonylamino)-2-morpholin-4-ylpyrimidin-4-yloxy]-ethyl ester,
pyridin-2-ylcarbamic acid 2-[5-(2-methoxy-phenoxy)-6-(5-methyl-pyridin-2-ylsulphonylamino)-2,2'-bipyridimin-4-yloxy]-ethyl ester,
pyridin-2-yl-carbamic acid 2-[5-(2-methoxy-phenoxy)-6-(5-methyl-pyridin-2-ylsulphonylamino)-2-morpholin-4-yl-pyrimidin-4-yloxy]-ethyl ester,
pyridin-2-ylcarbamic acid 2-[5-(2-chloro-5-methoxy-phenoxy)-6-(5-isopropylpyridin-2-sulphonylamino)-2-morpholin-4-yl-pyrimidin-4-yl-methoxy]-ethyl ester,
pyridin-2-ylcarbamic acid 5-(2-chloro-5-methoxy-phenoxy)-6-(5-isopropylpyridin-2-ylsulphonylamino)-2-morpholin-4-yl-pyrimidin-4-ylmethyl ester,
pyridin-2-ylcarbamic acid 2-[6-(2,5-dichlorothiophen-3-ylsulphonylamino)-5-(2-methoxy-phenoxy)-2,2'-bipyrimidin-4-yloxy]ethyl ester,
pyridin-2-ylcarbamic acid 2-[5-(2-methoxy-phenoxy)-6-(3,5-dimethyl-isoxazol-4-ylsulphonylamino)-2,2'-bipyrimidin-4-yloxy]ethyl ester,
pyridin-2-ylcarbamic acid 2-[6-(5-tert-butylthiophen-2-ylsulphonylamino)-5-(2-methoxy-phenoxy)-2-morpholin-4-yl-pyrimidin-4-yloxy]-ethyl ester,
pyridin-2-ylcarbamic acid 2-[6-(2,5-dichloro-thiophen-3-ylsulphonylamino)-5-(2-methoxy-phenoxy)-2-morpholin-4-yl-pyrimidn-4-yloxy]-ethyl ester,
pyridin-2-ylcarbamic acid 2-[6-(3,5-dimethyl-isoxazol-4-ylsulphonylamino)-5-(2-methoxy-phenoxy)-2-morpholin-4-yl-pyrimidin-4-yloxy]-ethyl ester.

10. Compounds according to claim 6 wherein $R^9$ is 4-pyridyl.

**11.** The compound according to claim 10,
pyridin-4-ylcarbamic acid 2-[6-(5-tert-hutyl-thiophen-2-ylsulphonylamino)-5-(2-chloro-5-methoxy-phenoxy)-pyrimidin-4-yloxy]-ethyl ester.

**12.** Compounds according to claim 5, wherein $R^3$ is a residue $-O(CH_2)_2-B-R^9$ and B is -(O)C(O)O-.

**13.** The compounds according to claim 12,

carboxylic acid furan-3-ylmethyl ester 2-(5-(2-methoxy-phenoxy)-6-(5-methylpyridin-2-sulphonylamino)-2-morpholin-4-yl-pyrimidin-4-yloxy] -ethyl ester,
5-isopropyl-pyridine-2-sulphonic acid (5-(2-methoxy-phenoxy)-2-morpholin-4-yl-6-[2-(pyrimidin-2-yloxy)-ethoxy]-pyrimidin-4-yl}-amide.

**14.** Compounds according to claim 5, wherein $R^3$ is a residue $-O(CH_2)_2-B-R^9$ and B is -NHC(O)NH-.

**15.** The compounds according to claim 14,

5-tert-butyl-thiophene-2-sulphonic acid 5-(2-methoxy-phenoxy) -6- [2- (3-pyridin-2-yl-ureido)-ethoxy]-2,2'-bipyrimidin-4-ylamide,
5-isopropyl-pyridine-2-sulphonic acid 5-(2-methoxy-phenoxy)-6-[2-(3-pyridin-2-yl-ureido)-ethoxy]-2,2'-bipyrimidin-4-ylamide.

**16.** Compounds according to claims 1-4, wherein $R^3$ is hydroxyethoxy.

**17.** The compounds according to claim 16,

5-tert-butyl-thiophene-2-sulphonic acid 6-(2-hydroxy-ethoxy)-5-(2-methoxy-phenoxy)-2,2'-bipyrimidin-4-ylamide,
5-pentyl-thiophene-2-sulphon ic acid 6-(2-hydroxy-ethoxy) -5-(2-methoxy-phenoxy)-2,2'-bipyrimidin-4-ylamide,
5-(2,2-dimethyl-propionyl)-thiophene-2-sulphonic acid 6-(2-hydroxy-ethoxy)-5-(2-methoxy-phenoxy)-2,2'-bipyrimidin-4-ylamide,
5-isopropyl-pyridine-2-sulphonic acid 6-(2-hydroxy-ethoxy)-5-(2-methoxy-phenoxy)-2,2'-bipyrimidin-4-ylamide,
pyridine-3-sulphonic acid 6-(2-hydroxy-ethoxy)-5-(2-methoxy-phenoxy)-2,2'-hipyrimidin-4-ylamide,
5-tert-butyl-thiophene-2-sulphonic acid 5-(2-chloro-5--methoxy-phenoxy)-6-(2-hydroxy-ethoxy)-pyrimidin-4-ylamide,
5-tert-butyl-thiophene-2-sulphonic acid 6-(2-hydroxy-ethoxy)-5-(2-methoxyphenoxy)-pyrimidin-4-ylamide,
N-[5-(2-chloro-5-methoxy-phenoxy)-6-(2-hydroxy-ethoxy)-pyrimidin-4-yl]-5-isopropyl-pyridine-2-sulphonamide,
5-isopropyl-N-[6-(2-hydroxy-ethoxy)-5-(2-methoxy-phenoxy)-2-(3-methoxyphenyl)-pyrimidin-4-yl]-pyridine-2-sulphonamide,
5-isopropyl-N-[6-(2-hydroxy-ethoxy)-5-(2-methoxy-phenoxy)-2-methylsulphanylpyrimidin-4-yl]-pyridine-2-sulphonamide,
N-[2-(1,3-benzodioxol-5-yl)-6-(2-hydroxy-ethoxy)-5-(2-methoxy-phenoxy)pyrimidin-4-yl]-5-isopropyl-pyridine-2-sulphonamide,
N-[5-(2-chloro-5-methoxy-phenoxy)-6-(2-hydroxy-ethoxy)-2-morpholin-4-yl-pyrimidin-4-yl]-5-isopropyl-pyridine-2-sulphonamide,
5-methyl-pyridine-2-sulphonic acid 6-(2-hydroxy-ethoxy)-5-(2-methoxy-phenoxy)-2,2'-bipyrimidin-4-ylamide,
5-methyl-pyridine-2-sulphonic acid 6-(2-hydroxy-ethoxy)-5-(2-methoxy-phenoxy)-2-morpholin-4-yl-pyrimidin-4-ylamide,
5-isopropyl-pyridine-2-sulphonic acid 6-(2-hydroxy-ethoxy)-5-(2-methoxyphenoxy)-2-morpholin-4-yl-pyrimidin-4-ylamide,
5-tert.butylthiophene-2-sulphonic acid 6-(2-hydroxy-ethoxy)-5-(2-methoxyphenoxy-2-morpholin-4-yl-pyrimidin-4-ylamide,
2,5-dichlorothiophene-3-sulphonic acid 6-(2-hydroxy-ethoxy)-5-(2-methoxyphenoxy)-2-morpholin-4-yl-pyrimidin-4-ylamide,
3,5-dimethylisoxazole-4-sulphonic acid 6-(2-hydroxy-ethoxy)-5-(2-methoxyphenoxy)-2-morpholin-4-yl-pyrimidin-4-ylamide,
2,5-dichlorothiophene-3-sulphonic acid 6-(2-hydroxy-ethoxy)-5-(2-methoxyphenoxy)-2,2'-bipyrimidin-4-ylamide,
3,5-dimethyl-isoxazole-4-sulphonic acid 6-(2-hydroxy-ethoxy)-5-(2-methoxyphenoxy)-2,2'-bipyrimidin-4-ylamide,
5-isopropyl-pyridine-2-sulphonic acid [6-(2-hydroxy-ethoxy)-2-(3-methoxybenzyl)-5-(2-methoxy-phenoxy)-pyrimidin-4-yl]-amide,
5-isopropyl-pyridine-2-sulphonic acid [6-(2-hydroxy-ethoxy)-2-(3-methoxybenzyl)-5-phenoxy-pyrimidin-4-yl]-amide,
5-isopropyl-pyridine-2-sulphonic acid [2-(3-hydroxy-benzyl)-6-(2-hydroxyethoxy)-5-phenoxy-pyrimidin-4-yl]-amide.

**18.** Compounds according to claims 1-4, wherein $R^3$ is aminoethoxy.

**19.** The compound according to claim 18,
5-tert-butyl-thiophene-2-sulphonic acid 6-(2-amino-ethoxy)-5-(2-methoxyphenoxy)-2,2'-bipyrimidin-4-ylamide.

**20.** Compounds according to claims 1-4, wherein $R^3$ is $C_{1-7}$-alkyl, $C_{1-7}$-alkoxy, formyl, halo-$C_{1-7}$-alkyl, hydroxy- $C_{1-7}$-alkyl or $-CH_2O-A-C_{1-7}$-alkyl.

**21.** The compounds according to claim 20,

5-Isopropyl-pyridine-2-sulphonic acid 5-(2-chloro-5-methoxy-phenoxy)-6-methyl-2-morpholin-4-yl-pyrimidin-4-ylamide,
5-(2-chloro-5-methoxy-phenoxy)-6-formyl-2-morpholin-4-yl-pyrimidin-4-ylamide,
5-isopropyl-pyridine-2-sulphonic acid 5-(2-chloro-5-methoxy-phenoxy)-6-hydroxymethyl-2-morpholin-4-yl-pyrimidin-4-ylamide,
5-isopropyl-pyridine-2-sulphonic acid 5-(2-chloro-5-methoxy-phenoxy)-6-chloro-methyl-2-morpholin-4-yl-pyrimidin-4-ylamide,
5-(2-chloro-5-methoxy-phenoxy)-6-(2-hydroxy-ethoxymethyl)-2-morpholin-4-yl-pyrimidin-4-ylamide.

**22.** Compounds of the formula

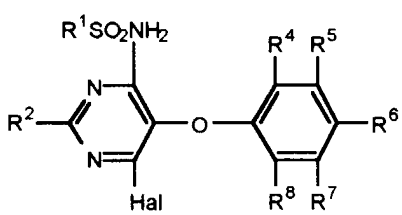

II

wherein $R^1$, $R^2$ and $R^4$-$R^8$ have the significance set forth in claim 1 and Hal is halogen.

**23.** The compounds of claims 1-21 for use as medicaments, especially for the treatment of disorders which are associated with endothelin activities, especially circulatory disorders such as hypertension, ischaemia, vasospasms and angina pectoris.

**24.** A process for the manufacture of compounds of claims 1-21, characterized by

a) reacting a compound of the formula

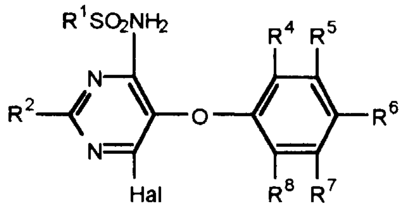

II

wherein $R^1$, $R^2$ and $R^4$-$R^8$ have the significance set forth above and Hal is halogen, with a compound of the formula

$$HO(CR^aR^b)_nXH$$

wherein n, $R^a$ and $R^b$ have the significance set forth above and X represents O or NH,
or

b) reacting a compound of the formula

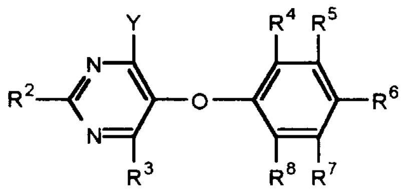

III

wherein $R^2$-$R^8$ have the significance set forth above,
with a compound of the formula

$$R^1SO_2Z$$

wherein $R^1$ has the significance set forth above and whereby Y represents halogen and Z represents amino or Y represents amino and Z represents halogen,
or

c) reacting a compound of the formula

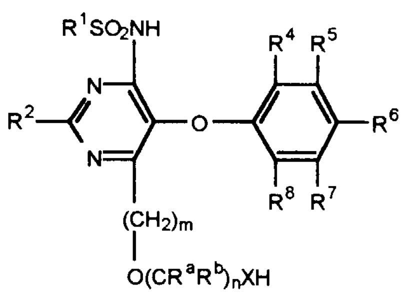

IV

wherein $R^1$, $R^2$, $R^4$-$R^8$, $R^a$, $R^b$, X, m and n have the significance set forth above,

c1) with an isocyanate of the formula $R^9NCO$ or a carbamoyl chloride of the formula $R^9NCOCl$, wherein $R^9$ has the significance set forth above, or

c2) with phosgene and thereafter with an alcohol of the formula $R^9OH$; or with a chloroformate of the formula $R^9OC(O)Cl$; or

d) reacting a compound of formula I in which $R^3$ represents halo-$C_{1-7}$-alkyl with a compound of the formula

$$HOCH_2\text{-A-}C_{1-7}\text{-alkyl}$$

wherein A represents a ketalized 1,2-dihydroxy-ethylene group,

and, if desired, modifying substituents present in the resulting compound of formula I and/or converting the compound of formula I obtained into a salt.

**25.** Pharmaceutical preparations, containing a compound of claims 1-21 and usual carriers and adjuvants.

**26.** Compounds according to any one of claims 1-21, when manufactured according to the process of claim 23.

**27.** The use of compounds of claims 1-21 as active substances for the production of medicaments for the treatment of disorders which are associated with endothelin activities, especially circulatory disorders such as hypertension, ischaemia, vasospasms and angina pectoris.

**28.** Compounds according to claims 1-21 for use in a method for the treatment of disorders which are associated with endothelin activities, especially circulatory disorders such as hypertension, ischaemia, vasospasms and angina pectoris, by administration of an effective amount of a compound of claims 1-21 to a person in need of such treatment.

**Revendications**

**1.** Composés de formule I :

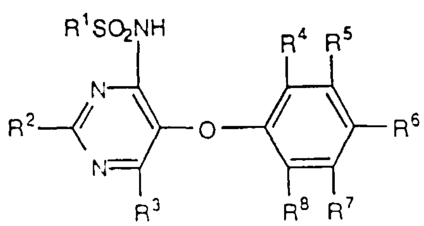

(I)

dans laquelle

$R^1$ est un radical hétérocyclique mono- ou bicyclique, contenant en tant qu'hétéroatome de l'oxygène, de l'azote ou du soufre, choisi parmi les groupes 2-furyle, 3-furyle, pyrimidinyle, 2-pyridiyle, 3-pyridyle, 4-pyridyle, 1,2-diazinyle, 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinyle, quinoléyle, isoquinoléyle et quinazolyle ; ou un radical hétérocyclique tel que ci-dessus, mono- ou di-substitué par des substituants alkyle en $C_{1-7}$, alcanoyle en $C_{1-7}$, halogéno, amino, monoalkylamino en $C_{1-7}$, di(alkyle en $C_{1-7}$)amino, ou par un autre radical hétérocyclique tel que ci-dessus ;

$R^2$ est un atome d'hydrogène, un groupe alkyle en $C_{1-7}$, alcoxy en $C_{1-7}$, alkylthio en $C_{1-7}$, (alcoxy en $C_{1-7}$)(alkyle en $C_{1-7}$), (alkyle en $C_{1-7}$)sulfonyl(alcoxy en $C_{1-7}$), phényle, (alkyle en $C_{1-7}$)phényle, (alcoxy en $C_{1-7}$)phényle, (alkylène en $C_{1-7}$)dioxyphényle, phényl(alkyle en $C_{1-7}$), (alkyle en $C_{1-7}$)phényl(alkyle en $C_{1-7}$), (alcoxy

en $C_{1-7}$) phényl(alkyle en $C_{1-7}$), (alkylène en $C_{1-7}$)dioxyphényl(alkyle en $C_{1-7}$), ou un radical hétérocyclique, mono- ou bicyclique, avec comme hétéroatome de l'oxygène, de l'azote ou du soufre choisi parmi les groupes 2-furyle, 3-furyle, pyrimidinyle, 2-pyridiyle, 3-pyridyle, 4-pyridyle, 1,2-diazinyle, 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinyle, thiomorpholino, pipéridino, pyrrolidino, benzodioxolyle, quinoléyle, isoquinoléyle et quinazolyle ; ou un radical hétérocyclique tel que ci-dessus, mono- ou disubstitué par des substituants alkyle en $C_{1-7}$, alcanoyle en $C_{1-7}$, halogéno, ou par un autre radical hétérocyclique tel que ci-dessus ; ou un groupe hétérocyclyle(alkyle en $C_{1-7}$), où le groupe hétérocyclyle est tel que défini ci-dessus ;

$R^3$ est un groupe alkyle en $C_{1-7}$, alcoxy en $C_{1-7}$, formyle, halogènalkyle en $C_{1-7}$, hydroxyalkyle en $C_{1-7}$, aminoalkyle en Ci-7 ou un radical $-CH_2O$-A-(alkyle en $C_{1-7}$), $-(CH_2)_m-O-(CR^aR^b)_nOH$, $-(CH_2)_m-O-(CR^aR^b)_nOR^9$, $-(CH_2)_m-O-(CR^aR^b)n-NH_2$ ou $-(CH_2)_m-O-(CR^aR^b)_n-B-R^9$ ;

$R^4$-$R^8$ sont des atomes d'hydrogène ou des groupes alcoxy en $C_{1-7}$ ou halogéno ;

$R^9$ est un radical hétérocyclique mono- ou bicyclique avec comme hétéroatome de l'oxygène, de l'azote ou du soufre, choisi parmi les groupes 2-furyle, 3-furyle, pyrimidinyle, 2-pyridiyle, 3-pyridyle, 4-pyridyle, 1,2-diazinyle, 1,4-diazinyle, morpholino, 2-thiényle, 3-thiényle, isoxazolyle, oxazolyle, thiazolyle, imidazolyle, pyrrolyle, pyrazinyle, benzofurannyle, benzothiényle, indolyle, purinyle, quinoléyle, isoquinoléyle et quinazolyle ; ou un radical hétérocyclique tel que ci-dessus, mono- ou disubstitué par des substituants alkyle en $C_{1-7}$, alcanoyle en $C_{1-7}$, halogéno, ou par un autre radical hétérocyclique tel que ci-dessus ; ou le groupe phé-nyle, ou un groupe phényle substitué par des groupes alkyle en $C_{1-7}$, alcoxy en $C_{1-7}$ et/ou halogéno, ou encore un groupe alkyle en $C_{1-7}$ ;

$R^a$ et $R^b$ sont des atomes d'hydrogène ou des groupes alkyle en $C_{1-7}$ ;

A est un groupe 1,2-dihydroxyéthylène cétalisé ;

B est -OC(O)O-, -OC(O)NH-, -NHC(O)NH- ou -NHC(O)O- ;

n vaut 2, 3 ou 4 ; et

m vaut 0 ou 1.

**2.** Composés selon la revendication 1, dans lesquels $R^2$ est un atome d'oxygène, un groupe alkyle en $C_{1-7}$, alcoxy en $C_{1-7}$, alkylthio en $C_{1-7}$, (alcoxy en $C_{1-7}$)-(alkyle en $C_{1-7}$), (alkyle en $C_{1-7}$)sulfonyl(alcoxy en $C_{1-7}$), phényle, (alkyle en $C_{1-7}$)-phényle, (alcoxy en $C_{1-7}$)phényle, (alkylène en $C_{1-7}$)dioxyphényle, ou un radical hétérocyclique, mono- ou bicyclique, avec comme hétéroatome de l'oxygène, de l'azote ou du soufre, choisi parmi les groupes 2-furyle, 3-furyle, pyrimidinyle, 2-pyridiyle, 3-pyridyle, 4-pyridyle, 1,2-diazinyle, 1,4-diazinyle, morpholino, 2-thiényle, 3-thiényle, isoxazolyle, oxazolyle, thiazolyle, imidazolyle, pyrrolyle, benzofurannyle, benzothiényle, indolyle, purinyle, thiomorpholino, pipéridino, pyrrolidino, benzodioxolyle, quinoléyle, isoquinoléyle et quinazolyle ; ou un radical hétérocyclique tel que ci-dessus, mono- ou disubstitué par des substituants alkyle en $C_{1-7}$, alcanoyle en $C_{1-7}$, halogéno, ou par un autre radical hétérocyclique tel que ci-dessus ; ou un groupe hétérocyclyle (alkyle en $C_{1-7}$), le groupe hétérocyclyle étant tel que défini ci-dessus ;
$R^3$ est un groupe alkyle en $C_{1-7}$, alcoxy en $C_{1-7}$, formyle, halogènalkyle en $C_{1-7}$, hydroxyalkyle en $C_{1-7}$, aminoalkyle en $C_{1-7}$ ou un radical $-CH_2O$-A-(alkyle en $C_{1-7}$), $-(CH_2)_m-O-(CR^aR^b)_nOH$, $-(CH_2)_m-O-(CR^aR^b)_nOR^9$, $-(CH_2)_m-O-(CR^aR^b)_n-NH_2$ ou $-(CH_2)_m-O-(CR^aR^b)_n-B-R^9$ ; et $R^1$, $R^4$, $R^9$, $R^a$, $R^b$, A, B n et m ont les significations données dans la revendication 1.

**3.** Composés selon la revendication 1 ou 2, dans lesquels R' est un groupe pyridyle, pyrimidinyle, isoxazolyle, furyle ou thiényle, non-substitué, ou substitué par des groupes alkyle en $C_{1-7}$, halogéno, amino, mono- ou di(alkyle en $C_{1-7}$)amino ou alcanoyle en $C_{1-7}$.

**4.** Composés selon l'une des revendications 1 à 3, dans lesquels $R^2$ est un atome d'hydrogène ou un groupe pyrimidinyle, pyridyle, morpholino, thiomorpholino, pipéridino, pyrrolidino, benzodioxyolyle, (alcoxy en $C_{1-7}$)-phényle ou alkylthio en $C_{1-7}$.

**5.** Composés selon l'une des revendications 1 à 4, dans lesquels $R^3$ est un radical $-O(CR^aR^b)_nOH$, $-O(CR^aR^b)_nNH_2$

ou un radical $-O(CH_2)_2-B-R^9$, et $R^9$ est un groupe pyridyle, pyrimidinyle ou furyle.

6. Composés selon la revendication 5, dans lesquels $R^3$ est un radical - $O(CH_2)_2-B-R^9$ et B est (O)C(O)NH-.

7. Composés selon la revendication 6, dans lesquels $R^9$ est le groupe 2-pyridyle.

8. Le composé selon la revendication 7 : ester 2-[6-(5-isopropylpyridine-2-ylsulfonylamino)-5-(2-méthoxyphénoxy)-2-morpholine-4-ylpyrimidine-4-yloxy]-éthylique de l'acide pyridine-2-ylcarbamique.

9. Les composés selon la revendication 7 :

ester 2-[6-(5-tertbutylthiophène-2-ylsulfonylamino)-5-(2-méthoxyphénoxy)-2, 2'-bipyrimidine-4-yloxy]éthylique de l'acide pyridine-2-ylcarbamique,

ester 2-[5-(2-méthoxyphénoxy)-6-(5-pentylthiophène-2-ylsulfonylamino)-2,2'-bipyrimidine-4-yloxy]-éthylique de l'acide pyridine-2-ylcarbamique,

ester 2-[6-(5-(2,2-diméthylpropionyl)-thiophène-2-ylsulfonylamino)-5-(2-méthoxyphénoxy)-2,2'-bipyrimidine-4-yloxy]éthylique de l'acide pyridine-2-yl-carbamique,

ester 2-[6-(5-isopropylpyridine-2-ylsulfonylamino)-5-(2-méthoxyphénoxy)-2, 2'-bipyrimidine-4-yloxy]éthylique de l'acide pyridine-2-ylcarbamique,

ester 2-[5-(2-méthoxyphénoxy)-6-(pyridine-2-ylsulfonylamino)-2,2'-bipyrimidine-4-yloxy]-éthylique de l'acide pyridine-2-ylcarbamique,

ester 2-[5-(2-méthoxyphénoxy)-6-pyridine-3-(ylsulfonylamino)-2,2'-bipyrimidine-4-yloxy]-éthylique de l'acide pyridine-2-ylcarbamique,

ester 2-[6-(5-tertbutylthiophène-2-ylsulfonylamino)-5-(2-chloro-5-méthoxyphénoxy)-pyrimidine-4-yloxy]éthylique de l'acide pyridine-2-ylcarbamique,

ester 2-[6-(5-isopropylpyridine-2-ylsulfonylamino)-5-(2-méthoxyphénoxy)-2-(3-méthoxyphényl)-pyrimidine-4-yloxy]-éthylique de l'acide pyridine-2-carbamique,

ester 2-[6-(5-isopropylpyridine-2-ylsulfonylamino)-5-(2-méthoxyphénoxy)-2-méthylsulfanylpyrimidine-4-yloxy]-éthylique de l'acide pyridine-2-carbamique,

ester 2-[2-(1,3-benzodioxol-5-yl)-6-(5-isopropylpyridine-2-ylsulfonylamino)-5-(2-méthoxyphénoxy)-pyrimidine-4-yloxy]éthylique de l'acide pyridine-2-carbamique,

ester 2-[5-(2-chloro-5-méthoxyphénoxy)-6-(5-isopropylpyridine-2-ylsulfonylamino)-2-morpholine-4-ylpyrimidine-4-yloxy]éthylique de l'acide pyridine-2-carbamique,

ester 2-[5-(2-méthoxyphénoxy)-6-(5-méthylthiophène-2-ylsulfonylamino)-2,2'-bipyrimidine-4-yloxy]-éthylique de l'acide pyridine-2-ylcarbamique,

ester 2-[5-(2-méthoxyphénoxy)-6-(5-méthylpyridine-2-ylsulfonylamino)-2-morpholine-4-ylpyrimidine-4-yloxy]-éthylique de l'acide pyridine-2-carbamique,

ester 2-[5-(2-chloro-5-méthoxyphénoxy)-6-(5-isopropylpyridine-2-sulfonylamino)-2-morpholine-4-ylpyrimidine-4-yl-méthoxy]éthylique de l'acide pyridine-2-carbamique,

ester 5-(2-chloro-5-méthoxyphénoxy)-6-( 5-isopropylpyridine-2-sulfonylamino)-2-morpholine-4-ylpyrimidine-4-ylméthylique de l'acide pyridine-2-carbamique,

ester 2-[6-(2,5-dichlorothiophène-3-ylsulfonylamino)-5-(2-méthoxyphénoxy)-2,2'-bipyrimidine-4-yloxy]éthylique de l'acide pyridine-2-yl-carbamique,

ester 2-[5-(2-méthoxyphénoxy)-6-(3,5-diméthylisoxazole-4-ylsulfonylamino)-2,2'-bipyrimidine-4-yloxy]-éthylique de l'acide pyridine-2-ylcarbamique,

ester 2-[6-(5-tertbutylthiophène-2-ylsulfonylamino)-5-(2-méthoxyphénoxy)-2-morpholine-4-ylpyrimidine-4-yloxy]éthylique de l'acide pyridine-2-yl-carbamique,

ester 2-[6-(2,5-dichlorothiophène-3-ylsulfonylamino)-5-(2-méthoxy-phénoxy)-2-morpholine-4-ylpyrimidine-4-yloxy]éthylique de l'acide pyridine-2-yl-carbamique,

ester 2-[6-(3,5-diméthylisoxazole-4-ylsulfonylamino)-5-(2-méthoxyphénoxy)-2-morpholine-4-ylpyrimidine-4-yloxy]éthylique de l'acide pyridine-2-yl-carbamique.

**10.** Composés selon la revendication 6, dans lesquels $R^9$ est le groupe 4-pyridyle.

**11.** Le composé selon la revendication 10: ester 2-[6-(5-tertbutylthiophène-2-ylsulfonylamino)-5-(2-chloro-5-méthoxy-phénoxy)-pyrimidine-4-yloxy]-éthylique de l'acide pyridine-2-ylcarbamique.

**12.** Composés selon la revendication 5, dans lesquels $R^3$ est un radical $-O(CR_2)_2-B-R^9$ et B-(O)C(O)O-.

**13.** Les composés selon la revendication 12 :

ester furanne-3-ylméthylique et ester 2-[5-(2-méthoxyphénoxy)-6-(5-méthylpyridine-2-sulfonylamino)-2-morpholine-4-ylpyrimidine-4-yloxy]-éthylique d'acide carboxylique,

{5-2-méthoxyphénoxy)-2-morpholine-4-yl-6-[2-(pyrimidine-2-yloxy)-éthoxy]-pyrimidine-4-yl}-amide de l'acide 5-isopropylpyridine-2-sulfonique.

**14.** Composés selon la revendication 5, dans lesquels $R^3$ est un radical $-O(CR_2)_2-B-R^9$ et B est NHC(O)NH-.

**15.** Les composés selon la revendication 14 :

5-(2-méthoxyphénoxy)-6-[2-(3-pyridine-2-yluréido)-éthoxy]-2,2'-bipyrimidine-4-ylamide de l'acide 5-tertbutylthiophène-2-sulfonique,

5-(2-méthoxyphénoxy)-6-[2-(3-pyridine-2-yluréido)-éthoxy]-2,2'-bipyrimidine-4-ylamide de l'acide 5-isopropylpyridine-2-sulfonique,

**16.** Composés selon les revendications 1 à 4, dans lesquels $R^3$ est le groupe hydroxyéthoxy.

**17.** Les composés selon la revendication 16 :

6-(2-hydroxyéthoxy)-5-(2-méthoxyphénoxy)-2,2'-bipyrimidine-4-ylamide de l'acide 5-tertbutylthiophène-2-sulfonique,

6-(2-hydroxyéthoxy)-5-(2-méthoxyphénoxy)-2,2'-bipyrimidine-4-ylamide de l'acide 5-pentylthiophène-2-sulfonique,

6-(2-hydroxyéthoxy)-5-(2-méthoxyphénoxy)-2,2'-bipyrimidine-4-ylamide de l'acide 5-(2,2-diméthylpropionyl)-thiophène-2-sulfonique,

6-(2-hydroxyéthoxy)-5-(2-méthoxyphénoxy)-2,2'-bipyrimidine-4-ylamide de l'acide 5-isopropylpyridine-2-sulfonique,

6-(2-hydroxyéthoxy)-5-(2-méthoxyphénoxy)-2,2'-bipyrimidine-4-ylamide de l'acide pyridine-3-sulfonique,

5-(2-chloro-5-méthoxyphénoxy)-6-(2-hydroxyéthoxy)-pyrimidine-4-ylamide de l'acide 5-tertbutylthiophène-2-sulfonique,

6-(2-hydroxyéthoxy)-5-(2-méthoxyphénoxy)-pyrimidine-4-ylamide de l'acide 5-tertbutylthiophène-2-sulfonique,

N-[5-(2-chloro-5-méthoxyphénoxy)-6-(2-hydroxyéthoxy)-pyrimidine-4-yl]-5-isopropylpyridine-2-sulfonamide,

5-isopropyl-N-[6-(2-hydroxyéthoxy)-5-(2-méthoxyphénoxy)-2-( 3-méthoxyphényl)-pyrimidine-4-yl]-pyridine-2-sulfonamide,

5-isopropyl-N-[6-(2-hydroxyéthoxy)-5-(2-méthoxyphénoxy)-2-(3-méthylsulfanyl)-pyrimidine-4-yl]-pyridine-2-sulfonamide,

N-[2-(1,3-benzodioxol-5-yl)-6-(2-hydroxyéthoxy)-5-(2-méthoxyphénoxy)-pyrimidine-4-yl]-5-isopropylpyridine-2-sulfonamide,

N-[5-(2-chloro-5-méthoxyphénoxy)-6-(2-hydroxyéthoxy)-2-morpholine-4-yl-pyrimidine-4-yl]-5-isopropylpyridine-2-sulfonamide,

6-(2-hydroxyéthoxy)-5-(2-méthoxyphénoxy)-2,2'-bipyrimidine-4-ylamide de l'acide 5-méthylpyridine-2-sulfonique,

6-(2-hydroxyéthoxy)-5-(2-méthoxyphénoxy)-2-morpholine-4-ylpyrimidine-4-ylamide de l'acide 5-méthylpyridine-2-sulfonique,

6-(2-hydroxyéthoxy)-5-(2-méthoxyphénoxy)-2,2'-bipyrimidine-4-ylamide de l'acide 5-isopropylpyridine-2-sulfonique,

6-(2-hydroxyéthoxy)-5-(2-méthoxyphénoxy)-2,2'-bipyrimidine-4-ylamide de l'acide 5-tertbutylthiophène-2-sulfonique,

6-(2-hydroxyéthoxy)-5-(2-méthoxyphénoxy)-2,2'-bipyrimidine-4-ylamide de l'acide 2,5-dichlorothiophène-3-sulfonique,

6-(2-hydroxyéthoxy)-5-(2-méthoxyphénoxy)-2,2'-bipyrimidine-4-ylamide de l'acide 3,5-diméthylisoxazole-4-sulfonique,

6-(2-hydroxyéthoxy)-5-(2-méthoxyphénoxy)-2,2'-bipyrimidine-4-ylamide de l'acide 2,5-dichlorothiophène-3-sulfonique,

6-(2-hydroxyéthoxy)-5-(2-méthoxyphénoxy)-2,2'-bipyrimidine-4-ylamide de l'acide 3,5-diméthylisoxazole-4-sulfonique,

[6-(2-hydroxyéthoxy)-2-(3-méthoxybenzyl)-5-(2-méthoxyphénoxy)-pyrimidine-4-yl]-amide de l'acide 5-isopropylpyridine-2-sulfonique,

[6-(2-hydroxyéthoxy)-2-(3-méthoxybenzyl)-5-(2-phénoxy)-pyrimidine-4-yl]-amide de l'acide 5-isopropylpyridine-2-sulfonique,

[2-(3-hydroxybenzyl)-6-(2-hydroxyéthoxy)-5-phénoxypyrimidine-4-yl]-amide de l'acide 5-isopropylpyridine-2-sulfonique,

**18.** Composés selon les revendications 1 à 4, dans lesquels $R^3$ est le groupe aminoéthoxy.

**19.** Les composés selon la revendication 18: 6-(2-aminoéthoxy)-5-(2-méthoxyphénoxy)-2,2'-bipyrimidine-4-ylamide de l'acide 5-tertbutylthiophène-2-sulfonique,

**20.** Composés selon les revendications 1 à 4, dans lesquels $R^3$ est un groupe alkyle en $C_{1-7}$, alcoxy en $C_{1-7}$, formyle, halogénalkyle en $C_{1-7}$, hydroxyalkyle en $C_{1-7}$ ou $-CH_2O-A$(alkyle en $C_{1-7}$).

**21.** Les composés selon la revendication 20 :

5-(2-chloro-5-méthoxyphénoxy)-6-méthyl-2-morpholine-4-ylpyrimidine-4-yl-amide de l'acide 5-isopropylpyridine-2-sulfonique,

5-(2-chloro-5-méthoxyphénoxy)-6-formyl-2-morpholine-4-ylpyrimidine-4-yl-amide,

5-(2-chloro-5-méthoxyphénoxy)-6-hydroxyméthyl-2-morpholine-4-yl-pyrimidine-4-ylamide de l'acide 5-isopropylpyridine-2-sulfonique,

5-(2-chloro-5-méthoxyphénoxy)-6-chlorométhyl-2-morpholine-4-yl-pyrimidine-4-ylamide de l'acide 5-isopropylpyridine-2-sulfonique,

5-(2-chloro-5-méthoxyphénoxy)-6-(2-hydroxyéthoxyméthyl)-2-morpholine-4-ylpyrimidine-4-ylamide.

**22.** Composés de formule :

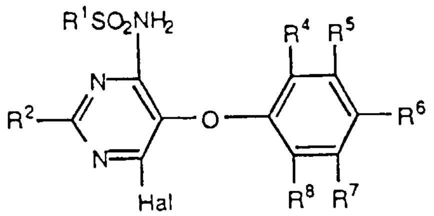

(II)

dans laquelle $R^1$, $R^2$ et $R^4$-$R^8$ ont les significations données dans la revendication 1, et Hal est un halogène.

**23.** Les composés selon les revendications 1 à 21, pour utilisation en tant que produits de soin, en particulier pour le traitement de maladies qui sont associées aux activités d'endothéline, en particulier les maladies de la circulation telles que l'hypertonie, l'ischémie, les angiospasmes et l'angine de poitrine.

**24.** Procédé de préparation de composés selon les revendications 1 à 21, caractérisé en ce que

a) on fait réagir un composé de formule

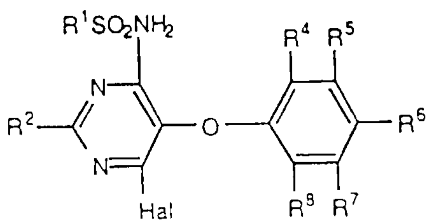

(II)

dans laquelle $R^1$, $R^2$ et $R^4$-$R^8$ ont les significations données ci-dessus et Hal est un halogène, avec un composé de formule

$$HO(CR^aR^b)_nXH$$

dans laquelle n, $R^a$ et $R^b$ ont les significations données ci-dessus, et X est O ou NH, ou

b) on fait réagir un composé de formule

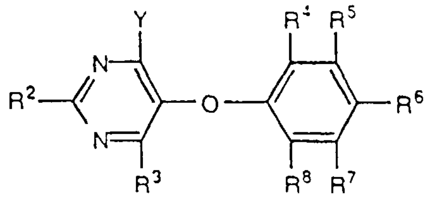

(III)

dans laquelle $R^2$-$R^8$ ont les significations données ci-dessus, avec un composé de formule

$$R^1SO_2Z$$

dans laquelle $R^1$ a les significations données ci-dessus, et Y est un halogène et Z est un groupe amino, ou encore Y est un groupe amino et Z est un halogène, ou

c) on fait réagir un composé de formule

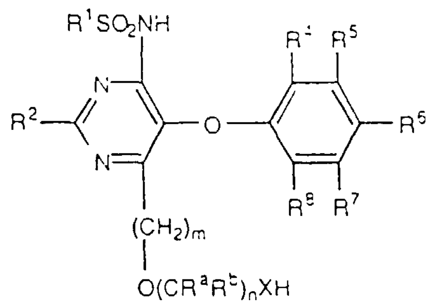

(IV)

dans laquelle $R^1$, $R^2$, $R^4$-$R^8$, $R^a$, $R^b$, X, m et n ont les significations données ci-dessus,

c1) avec un isocyanate de formule $R^9NCO$ ou un chlorure de carbamoyle de formule $R^9NCOCl$, où $R^9$ a les significations données ci-dessus, ou

c2) avec du phosgène puis avec un alcool de formule $R^9OH$ ; ou avec un chloroformiate de formule $R^9OC(O)Cl$ ; ou

d) on fait réagir un composé de formule I dans laquelle $R^3$ est un groupe halogènalkyle en Ci-7 avec un composé de formule

$$HOCH_2\text{-A(alkyle en }C_{1-7})$$

dans laquelle A est un groupe 1,2-dihydroxyéthylène cétalisé,

et on modifie les substances éventuellement contenues dans le composé obtenu de formule I, et/ou on convertit en un sel le composé obtenu de formule I.

**25.** Préparations pharmaceutiques contenant un composé selon les revendications 1 à 21 et des excipients et adjuvants usuels.

**26.** Composés selon les revendications 1 à 21, quand ils sont préparés par le procédé selon la revendication 23.

**27.** Utilisation de composés selon les revendications 1 à 21 en tant que principes actifs lors de la préparation de

produits de soins pour le traitement de maladies associées à des activités d'endothéline, en particulier les maladies de la circulation telles que l'hypertonie, l'ischémie, les angiospasmes et l'angine de poitrine.

**28.** Composés selon les revendications 1 à 21 pour utilisation dans un procédé pour le traitement de maladies qui sont associées à des activités d'endothéline, en particulier les maladies de la circulation telles que l'hypertonie, l'ischémie, les angiospasmes et l'angine de poitrine, par administration, à une personne ayant besoin d'un tel traitement, d'une quantité efficace d'un composé selon les revendications 1 à 21.